# EUROPÄISCHE PATENTSCHRIFT

(11) **EP 2 779 891 B1**
(45) Veröffentlichungstag und Bekanntmachung des Hinweises auf die Patenterteilung: **10.02.2016**
(21) Anmeldenummer: 12797784.1
(22) Anmeldetag: 16.11.2012
(51) Int. Cl.: A61B 5/00

(54) **HAUTKONTAKTERKENNUNGSVORRICHTUNG FÜR EIN ZU SICHERNDES GERÄT**
SKIN CONTACT RECOGNITION DEVICE FOR SAFEGUARDING AN APPARATUS
DISPOSITIF DE DETECTION DE CONTACT AVEC LA PEAU POUR SAUVEGARDER UN APPARAIL

(30) Priorität: 16.11.2011 AT 17042011; 17.10.2012 US 201261795454 P
(43) Veröffentlichungstag der Anmeldung: 24.09.2014
(73) Patentinhaber: Pantec Biosolutions AG, 9491 Ruggell (LI)
(72) Erfinder: BRAGAGNA, Thomas, FL-9493 Mauren (LI); HAGEN, Clemens, A-6841 Mäder (AT); HEINRICH, Arne, A-6800 Feldkirch (AT); KRAMMER, Peter, CH-9470 Buchs (CH); SUMMER, Stefan, A-6835 Dafins (AT)
(74) Vertreter: Burger, Hannes
(86) Internationale Anmeldenummer: PCT/EP2012/072860
(87) Internationale Veröffentlichungsnummer: WO 2013/072472

(56) Entgegenhaltungen:
- EP-B1- 2 054 193
- US-B2- 6 976 984
- US-B2- 7 842 029

## Beschreibung

Die Erfindung betrifft eine Hautkontakt-Erkennungsvorrichtung.

Für eine Vielzahl von Geräten ist eine eindeutig festgelegte Arbeitsumgebung von entscheidender Bedeutung, um eine Gefährdung von handelnden bzw. zu behandelnden Personen zu verhindern. Beispielsweise kann die Wirkweise des Geräts darin liegen, dass eine potentiell gesundheitsgefährdende Energieform abgegeben wird, um damit eine Wirkung auf der Hautoberfläche bzw. auf biologischem Gewebe zu erreichen. Die abgegebene Energie kann beispielsweise eine Hochenergie-Laserstrahlung sein, insbesondere eine in Laserklasse 4 eingeordnete Laserstrahlung, welche bei direktem Kontakt mit dem Auge zu irreversiblen Schädigungen des Auges führen würde. Auch kann ein unkontrolliertes Auftreffen einer derartigen Strahlung auf der Haut zu massiven Schädigungen des Hautgewebes führen.

Bislang war bekannt, dass derartige Systeme nur unter besonders geschützten Umgebungsbedingungen in Betrieb genommen werden durften, beispielsweise mussten derartige Geräte in einem zutrittsgesicherten Raum aufgestellt und betrieben werden, wobei alle Personen während des Betriebs des Geräts jedenfalls Schutzbrillen und gegebenenfalls Schutzkleidung tragen mussten. Auch war eine Bedienung bzw. Anwendung eines derartigen Geräts nur explizit ausgebildeten Spezialisten vorbehalten, was einer breiten Verbreitung derartiger Geräte im Wege steht, insbesondere ist bei derartigen Geräten eine Anwendung im Heimbereich bzw. außerhalb von Spezialeinrichtungen undenkbar.

Im Hinblick auf die Gefährdung wurde speziell für Lasersystem eine Klassifizierung eingeführt (nach EN 60825-1), wobei im Wesentlichen lediglich die Laserklasse 1 für eine allgemeine Verwendung zugelassen ist, da bei einem Gerät der Laserklasse 1 sichergestellt ist, dass eine gegebenenfalls austretende Laserstrahlung, auch bei direkter Einwirkung auf das Auge bzw. auf die Haut, zu keiner Schädigung derselben führen kann.

Ein weiterer Aspekt der Erfindung betrifft Geräte bzw. Vorrichtungen, welche bei Inbetriebnahme gesundheitsgefährdende Stoffe bzw. Partikel abgeben können. Beispielsweise betrifft dies jede Art von explosiv bzw. chemisch oder biologisch aktiven Vorrichtungen, insbesondere Sprengvorrichtungen und Feuerwaffen sowie Freigabevorrichtungen für biologische und/oder chemische Stoffe.

Bei beiden beschriebenen Verwendungsszenarien ist es daher von entscheidender Bedeutung, dass vor Inbetriebnahme bzw. während des Betriebs sichergestellt werden kann, dass die zu sichernde Vorrichtung direkten Kontakt mit biologischem Gewebe hat, insbesondere dass ein Hautkontakt hergestellt ist.

Aus dem Stand der Technik sind Sicherungssysteme bekannt, welche elektrische Kenngrößen von biologischem Gewebe ermitteln und darüber einen Hautkontakt feststellen. Beispielsweise zeigt die EP 1 898 825 A2 eine Vorrichtung, welche die Impedanz des biologischen Gewebes ermittelt und darüber eine Hautkontaktierung erkennt. Derartige Systeme haben allerdings den Nachteil, dass sie recht leicht umgangen werden kann, beispielsweise in dem an den Kontaktelektroden ein Widerstandsnetzwerk angelegt und somit ein biologisches Gewebe simuliert wird. Für Anwendungen im Medizinbereich hat eine elektrische Kontaktierung einer Person den weiteren Nachteil, dass sehr hohe Sicherheitsauflagen zu erfüllen sind, wenn eine Person leitend in Kontakt mit einem Gerät kommt, welches mit dem Energieversorgungsnetz verbunden ist. Auch würde eine derartige elektrische Kontaktierung versagen, wenn auf der Oberfläche der Haut eine Schutzschicht bzw. eine Abdeckschicht angebracht ist.

Ferner sind aus dem Stand der Technik kraftbasierte Hautkontakt-Erkennungsvorrichtungen bekannt, bei denen ein Hautabschnitt mit einer bestimmten, mechanischen Kraft auf ein Kontaktstück drücken muss, um die zu sichernde Vorrichtung in Betrieb nehmen zu können. Derartige Vorrichtungen, bspw. die EP1 460 955 A1, sind jedoch auch sehr einfach zu umgehen, da ein Betätigungselement geschaffen werden kann, welches das Kontaktstück mit der geforderten Kraft beaufschlag und somit einen Hautkontakt simuliert und die zu sichernde Vorrichtung unberechtigterweise in Betrieb nimmt.

Aus der US 7,413,567 B2 ist ferner eine Vorrichtung bekannt, welche die Reflexion unterschiedlicher ausgesandter Wellenlängen erfasst und durch Vergleich mit zuvor ermittelten Referenzwerten ein Aufliegen des Sensors auf der Haut feststellt. Zur Feststellung des Vorhandenseins von Haut wird der Sensor zuerst auf die Haut aufgelegt und durch Bestätigung der Dunkelwert ermittelt, welcher für die weiteren Messungen als Referenz verwendet wird. Anschließend wird, bei abgehobenem Sensor, hintereinander Licht mit zumindest drei unterschiedlichen Wellenlängen abgegeben. Durch Differenzbildung des reflektierten Lichts mit dem Dunkelwert wird ein Wertefeld ermittelt, welches gegen Grenzwerte geprüft wird, um so eine Annäherung an die Haut zu ermitteln. Von Nachteil ist hier, dass zuerst ein Dunkelwert ermittelt werden muss, wobei hier bereits eine Manipulationsmöglichkeit gegeben ist. Der Dunkelwert wird bei diesem System durch eine Benutzeraktion festgelegt, vom Sensor besteht keine Möglichkeit das tatsächliche Aufliegen auf der Haut zweifelsfrei zu bestimmen. Es kann also auch eine Resthelligkeit durch einen ungenau aufgelegten Sensor als Dunkelwert festgelegt werden, sodass die anschließende Annäherungserkennung zu früh ein Aufliegen des Sensors auf der Haut erkennen würde.

Ein ähnliches System ist auch aus der US 8,098,900 B2 bekannt. Das Dokument offenbart eine Vorrichtung bei der mittels Licht zweier unterschiedlicher Wellenlängen das Vorhandensein von menschlicher Haut im Erfassungsbereich bestimmt wird. Auch hier wird zur Ermittlung eines Referenzwertes der Sensor auf die Haut aufgelegt, um daran anschließend, die Unterscheidung durchführen zu können.

Aus der EP 2 054 193 B1 ist ferner eine Sicherungsvorrichtung für eine Werkzeugmaschine bekannt, bei der eine Strahlungseinheit Licht zumindest zweier unterschiedlicher Wellenlängen in einen Erfassungsbereich abgibt und durch Auswerten des Reflektierten Lichts ermittelt, ob sich im Erfassungsbereich menschliches Gewebe, oder Material, Werkzeug oder Gegenstände befindet und erkanntem bei menschlichen Gewebe, einen Schutz aktiviert. Aus dem Stand der Technik bekannte Vorrichtungen funktionieren entweder auf dem Prinzip einer Kontaktierung (Kraft oder Elektrisch), oder erfordern die Bestimmung eines Referenzwertes bzw. erlauben lediglich eine qualitative Bestimmung des Vorhandenseins von Haut. Jedenfalls kann mit den bekannten Vorrichtungen die Hauterkennung zumeist recht einfach manipuliert werden. Kontakte können bspw. durch Haltemittel fixiert werden, eine elektrische Impedanzmessung kann durch Anbringen einer Ersatzimpedanz manipuliert werden. Bei kontaktlos wirkenden Systemen kann durch eine Manipulation bei der Festlegung des Referenzwertes die Sicherungsfunktionalität ausgehebelt werden. Im Hinblick auf die Sicherung von Geräten, die bei unsachgemäßen Handlungen umgebenden Personen einen Schaden zufügen können, muss jedoch sicher gestellt werden, dass ein Manipulationsversuch und damit ein vermeintlicher sicherer Betriebszustand der zu sichernden Vorrichtung erkannt bzw. verhindert wird.

Ferner ist aus der US 6,976,984 B2 eine Vorrichtung bekannt, um Haut mit hochenergetischer Strahlung, insbesondere Laserstrahlung, zu behandeln. Es ist eine Sicherungsvorrichtung vorhanden, welche eine Aktivierung der Laserstrahlungsquelle nur dann ermöglicht, wenn die Vorrichtung vollständig auf der Haut aufliegt. Insbesondere ist offenbart, dass Licht mit unterschiedlicher Wellenlänge auf die Haut gerichtet wird und das von der Haut reflektierte Licht von einem Detektor erfasst wird. Aufgrund der Zusammensetzung der einzelnen Hautschichten kommt es zu einer Wellenlängen-abhängigen Reflexion bzw. Absorption des von der Lichtquelle auf die Haut gelenkten Lichts. Durch die Ermittlung bzw. Auswertung eines Reflexions- bzw. Absorptionskoeffizienten kann sehr zuverlässig ermittelt werden, ob die Detektoröffnung vollständig von Haut bedeckt ist. Die Lichtquelle und der Lichtsensor sind derart in einem Gehäuse angeordnet, dass direktes Licht von der Lichtquelle nicht auf den Lichtsensor gelangen kann, sondern dass dies nur über Reflexion über die Haut möglich ist. Ungenügender oder fehlender Kontakt mit der Haut führt zu einer deutlichen Reduktion des Anteils des den Sensor erreichenden Lichtes, wodurch ein fehlender Hautkontakt ermittelt werden kann. Ferner ist offenbart, dass Licht von zwei Lichtquellen ausgesandt wird, wobei das von der Haut zurückreflektierte Licht von einem Photodetektor erfasst wird. Um die erfassten Anteile der jeweiligen Lichtquelle zuordnen zu können, ist offenbart, dass die Lichtquellen alternierend angesteuert werden, sodass vom Detektor sukzessive der Anteil der ersten und anschließend der Anteil der zweiten Lichtquelle erfasst wird.

Die Aufgabe der Erfindung liegt also darin ein Verfahren und eine Vorrichtung zu schaffen, welche ein eindeutiges Aufliegen eines Kontaktstückes auf biologischem Gewebe ermittelt, welche ohne elektrische und/oder mechanische Kontaktierungskomponenten und ohne einen Kalibrierungsschritt auskommt und mit der sich zuverlässig sicherstellen lässt, dass es sich um biologisches Gewebe handelt und dass der Kontakt hergestellt und gehalten wird. Es soll ferner verhindert werden, dass durch eine bewusste oder unbewusste Fehlkalibrierung eine Manipulation der Sicherungsfunktionalität möglich ist. Ferner muss die Vorrichtung kompakt ausgeführt sein und im Hinblick auf batterie- bzw. akkubetriebene Geräte einen möglichst geringen elektrischen Energieverbrauch aufweisen.

Die Aufgabe der Erfindung wird durch ein Verfahren zur Erkennung der Annäherung und des Aufliegens eines Geräts auf der Hautoberfläche gelöst. Dabei wird von der Lichtquelle eine erste und zweite ausgesandte Wellenlänge über den Lichtaustrittsabschnitt in Richtung der Hautoberfläche abgegeben. Die über den Lichteintrittsabschnitt den Fotodetektor erreichende erste und zweite Wellenlänge wird vom Fotodetektor erfasst und in ein, der Intensität proportionales, erstes und zweites erfasstes Signal umgewandelt. Von der Auswerteschaltung wird für das erste und zweite erfasste Signal ein erster und zweiter zeitlicher Signalverlauf gebildet, wobei von der Vergleichsschaltung der Auswerteschaltung der erste und zweite Signalverlauf mit den in der Datenbank hinterlegten Referenzdatensätzen verglichen wird. Ferner wird von der Auswerteschaltung ein Sicherer-Betriebszustand Signal dann abgegeben, wenn der erste und zweite erfasste Signalverlauf mit Referenzdaten für erste und zweite erfasste Signalverläufe übereinstimmt.

Nach einer Weiterbildung ist vorgesehen, dass der erste und zweite Signalverlauf mittels einer Glättungsfunktion ermittelt wird. Da dem erfassten ersten und zweiten Signal eine Vielzahl von Umgebungssignalen überlagert sein kann und die Annäherung an die Haut nicht notwendiger Weise mit konstanter Geschwindigkeit erfolgen muss, bspw. kann der Bediener geringfügig zittern, könnte es durch die Überlagerungen zu einer Überschreitung hinterlegter Grenzwerte kommen. Durch einen Glättungsvorgang können nun derartige, zumeist sehr kurzfristige Schwankungen, eliminiert werden, und der ermittelte Signalverlauf weist keine, oder nur vernachlässigbare Artefakte auf. Eine nicht abschließende Aufstellung derartiger Glättungsfunktionen umfasst bspw. einen gleitenden linearer Mittelwert, Spline-Interpolation, Gauß-Hüllkurve, Fensterfunktion, Normierung. Dem Fachmann sind hierzu weitere Funktionen bekannt, um für ein Signal mit überlagerten Störungen, die Störanteile weitestgehend zu eliminieren bzw. solche erfasste Signale der weiteren Verarbeitung nicht zuzuführen.

Ein Aufliegen des Kontaktstücks ist durch eine zumeist recht eindeutige Signalkonstellation des ersten und zweiten erfassten Signals gekennzeichnet, zumeist verschwindet das erste erfasste Signal beinahe vollständig. Bei einem Vergleich mit scharfen Grenzen würde bei Erfassung eines ersten erfassten Signals ein Nicht-Sicherer-Betriebszustand Signal abgegeben und die zu sichernde Vorrichtung deaktiviert werden. Nach einer Weiterbildung ist daher vorgesehen, dass die Vergleichsschaltung einen unscharfen Vergleich durchführt und neben der Übereinstimmung, zumindest ein zweites Betriebszustands-Signal abgibt, insbesondere ein Warnsignal. Somit lassen sich Abstandsbereiche definieren, in denen bspw. an den Bediener ein Warnsignal abgegeben wird, die Vorrichtung aber aktiv bleibt. Erst bei einem noch größeren Abstand wird dann das Nicht-Sicherer-Betriebszustand Signal abgegeben und die Vorrichtung desaktiviert. Wie bereits zuvor erwähnt, kann bei einer sehr geringen Abweichung vom vollständigen Aufliegen, bspw. im Bereich von 2mm bis zu 5mm, nur gestreute bzw. diffuse Laserstrahlung austreten, die keine Gesundheitsgefährdung hervorrufen kann. Der Bediener soll jedoch darüber Informiert werden, um die Kontaktvorrichtung korrekt auszurichten, um so eine Abschaltung zu verhindern. Gegebenenfalls kann zusätzlich ein Zeitgeber vorgesehen sein, der bei Erkennen eines Warnzustandes gestartet wird und die Vorrichtung deaktiviert, wenn der Warnzustand über einen zu langen Zeitraum bestehen bleibt. Bevorzugt wird der Warnzustand dem Bediener optisch und/oder akustisch signalisiert.

In Voruntersuchungen die zur gegenständlichen Erfindung geführt haben hat sich gezeigt, dass bei Annäherung an die Haut, die Pegel der ersten und zweiten erfassen Signale auf ein Maximum ansteigen, um dann entsprechend stark abzufallen. Es hat sich ableiten lassen, dass dieses Maximum für die gegenständliche Ausführung des Kontaktstücks bei ca. 10mm Abstand von der Hautoberfläche liegt. Mit einer anderen Ausführung des Kontaktstückes, insbesondere bei Änderung des Lichtaustritts- und Eintrittsabschnitts, kann sich dieser Wert selbstverständlich ändern. Wichtig ist, dass dieses Maximum vorhanden ist und als ausgezeichneter Referenzpunkt für die Abstandsbestimmung verwendet werden kann. Daher ist gemäß einer Weiterbildung vorgesehen, dass bei der Bildung der zeitlichen Signalverläufe, eine Maximalwertfindung durchgeführt wird, um dadurch einen bekannten Abstand festlegen zu können. Auch ist es möglich, dass dem erfassten ersten und zweiten Signal deutliche Störungen überlagert sein können, die direkten Einfluss auf die Bildung der Signalverläufe haben können. Daher kann nach einer Weiterbildung vorgesehen sein, dass bei der Bildung der zeitlichen Signalverläufe, jeweils eine Trendanalyse durchgeführt wird. Beispielsweise können mittels einer Trendanalyse statistische Ausreißer korrekt bewertet werden.

Dahingehend ist nach einer Weiterbildung vorgesehen, dass die ermittelten Trends mit in den Referenzdatensätzen hinterlegten Trends oder mit aus den Referenzdatensätzen ermittelten Referenztrends verglichen werden. Damit ist eine weitere Möglichkeit gegeben, um den Hauttyp und/oder eine korrekte Annäherung bzw. das Aufliegen erkennen zu können.

Eine ausgezeichnete Bedingung für das Aufliegen des Kontaktstücks auf der Haut ist gemäß einer Weiterbildung dadurch gekennzeichnet, dass beim Aufliegen des Geräts bzw. des Kontaktstücks auf der Haut näherungsweise kein erstes Signals erfasst wird. Näherungsweise kein Signal bedeutet, dass die Amplitude des ersten erfassten Signals deutlich unter der Amplitude des zweiten erfassten Signals liegt, oder dass die Amplitude des ersten erfassten Signals unter der Empfindlichkeitsgrenze des Fotodetektors liegt. Da die erste Wellenlänge kurzwellig ist, wird diese von den Melanozyten in der Haut absorbiert, so dass kaum bzw. kein Licht vom Lichtaustrittsbereich über die Haut in den Lichteintrittsbereich gelangt.

Untersuchungen zufolge erhält man den Maximalwert der erfassten ersten und zweiten Signale bei einem Abstand von ca. 10mm zwischen Haut und gegenständlicher Ausführun8des Kontaktstücks. Gemäß einer Weiterbildung werden die ermittelten Signalverläufe ab dem ermittelten Maximalwert linear interpoliert. Durch die Interpolation kann nun vom erfassten ersten und zweiten Signalwert auf den Abstand zwischen Kontaktstück und Haut rückgeschlossen werden, um somit sehr genaue Abstandsbereiche festlegen zu können, in denen ein sicherer Betriebszustand oder ein Warnzustand gegeben ist. Aufgrund des geringen Abstands ist eine lineare Interpolation zulässig, es könnte jedoch auch eine exponentielle Interpolation verwendet werden, bspw. eine quadratische.

Eine vorteilhafte Weiterbildung besteht darin, dass der Lichteintrittsabschnitt von einer Vorrichtung zur Erzeugung eines Differenzdrucks mit einem Unterdruck oder einem Überdruck beaufschlagt wird und der Differenzdruck von einer Druck-Messvorrichtung überwacht wird, und bei Überschreiten eines Druck-Grenzwertes des Differenzdrucks das Sicherer-Betriebszustand Signal und bei Unterschreiten eines Druck-Grenzwertes des Differenzdrucks das Nicht-Sicherer-Betriebszustand Signal abgegeben wird. Somit kann als zusätzliche Sicherungsmaßnahme auch die Dichtheit des Kontaktstücks gegenüber der Umgebung herangezogen werden.

Die Aufgabe der Erfindung wird auch durch eine Hautkontakt-Erkennungsvorrichtung für ein zu sicherndes Gerät gelöst, welche Hautkontakt-Erkennungsvorrichtung zur Durchführung des gegenständlichen Verfahrens ausgebildet ist. Insbesondere umfasst die Hautkontakt-Erkennungsvorrichtung ein Kontaktstück mit einer Auflagefläche, eine Sicherungsschaltung mit einer Lichtquelle, einen Fotodetektor und eine Auswerteschaltung, welche Auswerteschaltung mit dem Fotodetektor verbunden ist. Die Auflagefläche weist einen Lichtaustritts- und einen davon abgegrenzten Lichteintrittsabschnitt auf, ferner weist die Lichtquelle eine Hauptstrahlrichtung der abgegebenen elektromagnetischen Strahlung auf, wobei die Hauptstrahlrichtung in Richtung des Lichtaustrittsabschnitts ausgerichtet ist. Der Fotodetektor weist einen Erfassungsbereich für elektromagnetische Strahlung auf, welcher Erfassungsbereich in Richtung des Lichteintrittsabschnitts ausgerichtet ist. Die Lichtquelle zur Abgabe von Licht einer ersten und zumindest einer zweiten Wellenlänge ausgebildet, und der Fotodetektor ist für die erste und zweite Wellenlänge sensitiv und zur Umwandlung einer empfangenen ersten Wellenlänge in ein der erstes erfasstes Signal und der empfangenen zweiten Wellenlänge in ein zweites erfasstes Signal ausgebildet. Die Auswerteschaltung weist eine Datenbank auf, in der Referenzdatensätze mit Referenzdaten für erfasste erste und zweite Signale hinterlegt sind, wobei die Auswerteschaltung dazu ausgebildet ist, aus dem erfassten ersten und zweiten Signal, einen ersten und zweiten zeitlichen Signalverlauf zu bilden. Die Auswerteschaltung weist ferner eine Vergleichsschaltung auf, welche Vergleichsschaltung zum Vergleich des ersten und zweiten zeitlichen Signalverlaufs mit den in der Datenbank hinterlegten Referenzdatensätzen und zur Abgabe eines Sicherer-Betriebszustand Signal ausgebildet ist..

In einer Weiterbildung kann ferner vorgesehen sein, dass auch die Lichtquelle mit der Auswerteschaltung verbunden ist, um somit eine Korrelation zwischen dem ausgesandten und dem erfassten Licht zu ermöglichen. Dadurch kann in vorteilhafter Weise eine bessere Unterdrückung der Störeinflüsse durch Umgebungslicht erreicht werden.

Da die Ausbreitungsbedingung für Licht in der Haut bzw. in biologischem Gewebe stark von der Wellenlänge abhängt, kann durch Wahl zweier unterschiedlicher Wellenlängen das Aufsetzen des Kontaktstücks auf der Haut zuverlässig erkannt werden. Wird bspw. eine Wellenlänge so gewählt, dass diese vom Gewebe nur leicht abgeschwächt wird, während die andere Wellenlänge durch das Gewebe stark gedämpft wird. In der weiteren Beschreibung wird unter der zweiten Wellenlänge jene verstanden, die vom Gewebe nur stark abgeschwächt wird. Das Aufsetzen ist nun dadurch erkennbar, dass nach der Annäherung des Kontaktstückes an die Hautoberfläche und damit zumeist einem Ansteigen der erfassten Intensitäten der beiden Wellenlängen, bei Aufsetzen die erfasste Intensität der zweiten Wellenlänge stark abfällt, während die Intensität der ersten Wellenlänge nur gering abfällt. Abhängig vom absoluten Wert der zweiten Wellenlänge, kann diese vollständig vom Gewebe absorbiert werden, so dass keine Intensität der zweiten Wellenlänge erfasst wird.

Wird während des sicheren Betriebszustandes die gegenständliche Vorrichtung von der Haut abgehoben, wenn auch nur sehr gering, kommt es wieder zu einer Änderung der erfassten Signale, wodurch das Sichererer-Betriebszustand Signal deaktiviert bzw. nicht mehr bereitgestellt wird und die zu sichernde Vorrichtung sofort deaktiviert werden kann.

In der Beschreibung wird Haut, Hautoberfläche und biologisches Gewebe gleichbedeutend verwendet. Zur Beschreibung von Detailmerkmalen werden die jeweils passenden Detailbegriffe verwendet.

Ferner wird im Weiteren unter dem Begriff Sicherungsfunktion bzw. Abwandlungen davon verstanden, dass das Aufliegen des Kontaktstückes auf der Haut gewährleistet ist.

In einer Weiterbildung ist vorgesehen, dass die erste Wellenlänge im kleiner 470nm ist, insbesondere bei 450nm. Insbesondere liegt die erste Wellenlänge in Bereich des sichtbaren Blau bis zum beginnenden UV. Für diesen Wellenlängenbereich bzw. für diese Wellenlänge weist biologisches Gewebe eine sehr hohe Dämpfung auf, so dass Licht im Gewebe bereits nach sehr kurzer Strecke absorbiert wird. Je weniger Licht der ersten Wellenlänge nach dem Aufliegen der gegenständlichen Vorrichtung auf der Hautoberfläche in den Lichteintrittsabschnitt gelangt, desto stärker wird die Änderung in den erfassten Signalen ausfallen und umso eindeutiger ist daher das Erkennen des Aufliegens möglich. Die Wellenlänge liegt ferner ein einem Bereich in dem natürliches Licht einen geringen Anteil aufweist. Damit ist eine Umgehung der Sicherheitsfunktionalität durch Anordnen einer Lichtquelle neben der gegenständlichen Vorrichtung nicht möglich. Bezüglich der Ausbreitungsbedingungen unterschiedlicher Wellenlängen in biologischem Gewebe wird auf die entsprechende Fachliteratur verwiesen. Eine Wellenlänge von 450nm wird recht effizient von den Melanozyten in der Schicht zwischen Epidermis und Stratum Corneum (der Basalschicht) absorbiert und dient dem körpereigenen Schutz vor schädlicher UV-Strahlung. Darauf basiert die Bräunung beim Sonnenbaden. Von Fitzpatrick wurden sechs Hauttypen klassifiziert, von Typ I (Keltischer Typ, sehr helle Hautfarbe) bis zu Typ VI (dunkelbraune bis schwarze Haut); je dunkler der Hauttyp, desto stärker ist die Absorption. In einer Weiterbildung ist ferner vorgesehen, dass die zweite Wellenlänge im Bereich von 610nm bis 660nm, liegt, insbesondere bei 635nm. Dieser Wellenlängenbereich bzw. diese Wellenlänge hat den Vorteil, dass biologisches Gewebe hier einen geringen Dämpfungsfaktor aufweist. Licht das in das biologische Gewebe eingelenkt wird, breitet sich in diesem aus, so dass eine entsprechend große Auflagefläche gesichert werden kann.

Eine Weiterbildung liegt darin, dass das Kontaktstück eine der Kontaktfläche gegenüber liegende Anschlussfläche aufweist, wobei die Anschlussfläche zur Anordnung der Hautkontakt-Erkennungsvorrichtung an dem zu sichernden Gerät ausgebildet ist. Mit dieser Weiterbildung wird eine Vorrichtung geschaffen, welche in sich abgeschlossen die Sicherstellung des korrekten Aufliegens des Kontaktstücks auf dem biologischen Gewebe gewährleistet und somit an eine Vielzahl zu sichernder Geräte angeflanscht werden kann. Die Anschlussfläche kann ferner elektrische Kontaktfläche aufweisen, um die gegenständliche Vorrichtung mit elektrischer Energie zu versorgen und um das Sichererer-Betriebszustand Signal an das zu sichernde Gerät zu übermitteln.

Nach einer Weiterbildung ist der Lichtaustrittsabschnitt über einen ersten Durchbruch im Kontaktstück und/oder dass der Lichteintrittsabschnitt über einen zweiten Durchbruch im Kontaktstück mit der Anschlussfläche verbunden. Damit wird eine Ausbildung geschaffen, bei der das Kontaktstück lediglich die Durchleitung des Lichts von der Lichtquelle zum biologischen Gewebe und von diesem zurück zum Fotodetektor gewährleistet. Dies hat den Vorteil, dass das Kontaktstück als Einwegteil gebildet sein kann und somit auch den hohen Anforderungen im Medizinbereich genügt.

Eine Weiterbildung, nach der der zweite Durchbruch durch einen Lichtleiter gebildet ist hat den Vorteil, dass sich nur ein kleiner und gut abgegrenzter Abschnitt der Hautoberfläche im Lichteintrittsabschnitt befindet. Durch die Gestaltung der Endflächen des Lichtleiters im Lichteintrittsabschnitt kann die Einkoppelbedingung sehr genau festgelegt werden, so dass eine Manipulation der Hautkontakt-Erkennung, durch Anordnen einer Lichtquelle in der Umgebung des Kontaktstücks, erschwert bzw. verunmöglicht wird. Licht welches den Lichteintrittsabschnitt aus seitlicher Richtung erreicht, würde nicht in den Lichtleiter eingekoppelt und. Der Lichtleiter kann bspw. durch eine Kunststofffaser gebildet sein, bevorzugt ist eine PMMA-Faser mit einem Überzug aus flouriertem Polymer mit einer NA von 0.55 und einem Öffnungswinkel von 55°. Weitere mögliche Ausführungsformen sind hinsichtlich ihrer wellenleitenden Eigenschaften in den geforderten Wellenlängenbereichen zu untersuchen.

Um eine Manipulation der Sicherungsschaltung zu verhindern, kann eine Weiterbildung auch darin bestehen, dass die Sicherungsschaltung im zu sichernden Gerät angeordnet ist. Damit lässt sich gewährleisten, dass keine, die Schutzfunktion gewährleistenden Komponenten einem manipulativen Zugriffsversuch ausgesetzt sind. Dies ist insbesondere im Hinblick auf die dauerhafte Gewährleistung der Sicherungsfunktion von Vorteil. Da das Kontaktstück mit biologischem Gewebe in Kontakt kommt, ist diese Ausbildung ferner von Vorteil, da somit das Kontaktstück einem Reinigungs- bzw. Sterilisationsprozess unterzogen werden kann, ohne die Komponenten der Sicherungsschaltung durch einen solchen Prozess zu gefährden.

Da durch das Aufliegen des Kontaktstücks auf der Hautoberfläche wird diese ggf. deformiert, insbesondere in den Bereichen des Lichteintritts- bzw. Lichtaustrittsabschnitts. Das zu sichernde Gerät kann bspw. ein optisch wirkendes Element aufweisen, welches auf einen definierten Abstand zwischen dem Element und der Hautoberfläche angewiesen ist. Nach einer Weiterbildung kann vorgesehen sein, dass im Lichtaustrittsabschnitt eine Distanzhaltevorrichtung angeordnet ist. Somit kann die Deformation der Hautoberfläche ausgeglichen werden bzw. eine definierte Wölbung der Hautoberfläche vorgegeben werden.

Eine Weiterbildung, nach der die Lichtquelle durch zumindest eine Leuchtdiode oder zumindest eine Laserdiode gebildet ist hat den Vorteil, dass die Wellenlänge des abgegebenen Lichtes sehr gut und sehr genau einstellbar ist. Damit kann die Wellenlänge einerseits gut an das Dämpfungsniveau des biologischen Gewebes angepasst werden. Andererseits ist der Fotodetektor für die abgegebene Wellenlänge sensitiv, sodass somit eine Paarung geschaffen werden kann, die einen Manipulationsversuch wesentlich erschwert. Insbesondere kann damit ein Manipulationsversuch mit einer starken, breitbandigen Lichtquelle, welche im Nahbereich des Kontaktstücks angeordnet ist verhindert, bzw. ggf. auch als solcher erkannt werden. Eine Ausbildung der Lichtquelle als Laserdiode hat den Vorteil, dass eine Laserdiode einerseits ein sehr schmalbandiges Lichtspektrum abgibt und zusätzlich einen sehr gerichteten Lichtstrahl abgibt, wodurch eine sehr gute Ortsauflösung der Hautdetektion erreicht werden kann. Gerade für die erste Wellenlänge unterliegen Leuchtdioden der Einschränkung, dass sie für blaues und kurzwelligeres Licht nur eine recht geringe Leuchtdichte bereitstellen können. Hier haben Laserdioden den Vorteil, dass einerseits für kurzwelliges Blau Bauteile verfügbar sind und zusätzlich eine hohe Leuchtdichte bereitstellen können. Um einen größeren Oberflächenabschnitt beleuchten zu können, muss der Strahl einer Laserdiode entsprechend aufgeweitet werden.

Nach einer Weiterbildung ist die Lichtquelle im Lichtaustrittsabschnitt angeordnet was den Vorteil hat, dass damit eine sehr gute Kopplung des von der Lichtquelle ausgesandten Lichtes in das biologische Gewebe gegeben ist.

Eine vorteilhafte Weiterbildung liegt ferner darin, dass in der Auflagefläche zumindest zwei Kontaktelektroden angeordnet sind, die mit der Auswerteschaltung verbunden sind. Der Kontakt mit biologischem Gewebe kann auch über eine Messung elektrischer Kenngrößen erfolgen, bspw. durch eine Impedanzmessung oder durch Ermittlung dielektrischer Verschiebungsströme.

Im medizinischen Bereich gelten für netzgebundene elektrische Geräte, welche bspw. über Sensoren einen direkten elektrisch leitenden Kontakt mit einem Pateinten haben, sehr strenge Sicherheitsauflagen hinsichtlich der elektrischen Isolation gegenüber der Netzversorgung. Mit einer Weiterbildung, nach der zwischen den Kontaktelektroden und der Auswerteschaltung ein galvanisch getrennter Signalkoppler angeordnet ist, wird erreicht, dass eine Messung elektrischer Kenngrößen des aufliegenden Gewebes möglich wird, ohne dass ein direkter Kontakt des netzbetliebenen Geräts mit der Haut eines Patienten besteht.

Eine weitere Möglichkeit zur Erkennung eines korrekten Aufliegens des Kontaktstückes auf der Hautoberfläche erhält man gemäß einer Weiterbildung, nach der dass der Lichtaustrittsabschnitt und/oder der Lichteintrittsabschnitt mit einer Vorrichtung zur Erzeugung eines Differenzdrucks gegenüber der Umgebung verbunden ist. Beim Aufliegen des Kontaktstückes bilden der Lichtaustrittsabschnitt und/oder der Lichteintrittsabschnitt zusammen mit der Hautoberfläche zumeist einen im Wesentlichen luftdicht abgeschlossenen Raum bzw. einen Raum, mit einer gegenüber dem Umgebungsdruck eindeutigen Druckdifferenz. Somit kann durch Auswerten der Druckverhältnisse durch ein Druckerfassungsmodul der Auswerteschaltung das Aufliegen des Kontaktstücks dadurch erkannt werden, dass sich ein stabiler Differenzdruck einstellt und somit das Sicherer-Betriebszustand Signal abgegeben werden kann. Ein Abheben des Kontaktstückes ist analog dazu durch eine Änderung des zuvor erfassten Differenzdruck erkennbar. Da sich bereits bei einer geringen Fehlauflage des Kontaktstücks kein stabiler Differenzdruck ausbilden wird, bzw. wird die Vorrichtung zur Erzeugung eines Differenzdrucks einen erhöhten Arbeitsaufwand erfordern, lässt diese Weiterbildung eine feine aufgelöste Erfassung des korrekten Aufliegens zu.

Eine Weiterbildung besteht ferner darin, dass Fotodetektor zumindest für eine dritte Wellenlänge sensitiv ist, wobei diese Wellenlänge derart gewählt ist, dass sie im typischen Umgebungslicht in ausreichender Intensität vorkommt. Ferner soll sie vom Gewebe ausreichend stark gedämpft werden, so dass es beim bestimmungsgemäßen Einsatz nicht vorkommen kann, dass diese Wellenlänge vom Fotodetektor erfasst wird. Da der Einsatz des zu sichernden Geräts überwiegend nicht im Dunkeln stattfinden wird, wird das Einsatzgebiet zumeist hell erleuchtet sein. Wenn nun der Fotodetektor zumindest eine dritte Wellenlänge erfassen kann, kann somit ein Anheben des Geräts auch dadurch erkannt werden, dass plötzlich Komponenten des Umgebungslichts den Fotodetektor erreichen.

Ein Fotodetektor hat technologisch bedingt, einen gewissen Spektralbereich seiner Sensitivität. Da auch im Umgebungslicht Spektralkomponenten im Bereich der ersten und zweiten ausgesandte Wellenlänge vorkommen können, ist es möglich, dass diese vom Fotodetektor fälschlicherweise als von der Lichtquelle stammend erfasst werden könnten. Nach einer Weiterbildung ist daher vorgesehen, dass im Erfassungsbereich des Fotodetektors oder im Fotodetektor ein optisches Filterelement angeordnet ist. Das Filterelement kann nun derart ausgebildet sein, dass es eine sehr scharfkantige Durchlasscharakteristik aufweist, welche insbesondere auf die erste und zweite ausgesandte Wellenlänge abgestimmt werden kann. Somit lassen sich Komponenten des Umgebungslichtes, sowie von in manipulativer Absicht angeordneter Beleuchtungskörper, ausfiltern, so dass nur noch die reflektierte bzw. gestreute erste und zweite Wellenlänge erfasst wird. Das Filterelement kann nun bspw. im Lichteintrittsabschnitt angeordnet sein. Es ist jedoch auch möglich, dass das Filterelement direkt im Fotodetektor angeordnet ist, bspw. als Filterschicht am fotosensitiven Element.

Eine Weiterbildung besteht auch darin, dass die Vergleichsschaltung zustandsabhängig ausgebildet ist, insbesondere als state-machine. Somit kann ein Ablauf der Veränderung der erfassten Signale festgelegt werden, welcher auftreten muss, um einen sicheren Betriebszustand erkennen zu können. Insbesondere wird, wie der Name sagt, mit Zuständen gearbeitet, die eine Eingangsgröße einnehmen muss, um den hinterlegten Ablauf zu erfüllen. Dabei sind Absolutwerte ggf. nicht von Interesse, solange der gewünschte Trend eingehalten wird. Da sich die erfassten Signale je nach Hauttyp in ihrer Intensität deutlich unterscheiden werden, der grundsätzliche Intensitätsverlauf bei der Annäherung jedoch bei allen Hauttypen im Wesentlichen ähnlich sein wird, lässt sich mit einem zustandsabhängigem Vergleich, die Variabilität des Individualablaufs in den Griff bekommen.

Nach einer Weiterbildung weisen die Referenzdaten der Referenzdatensätze eine Hierarchie auf. Durch diese Weiterbildung wird erreicht, dass eine Abhängigkeit des Verlaufs der erfassten Signale gewährleistet werden kann. Insbesondere werden die absoluten Intensitätswerte der erfassten Signale vom Hauttyp abhängen. Da die Referenzdaten durch Messreihen an verschiedenen Hauttypen ermittelt wurden und als Absolutwerte in den Datensätzen interlegt wurden, kann beim Vergleich bspw. ermittelt werden, auf welchem Hauttyp die Annäherung erfolgt.

Nach einer Weiterbildung ist vorgesehen, dass die Referenzdaten der Referenzdatensätze als Paarungen von Bereichsangaben hinterlegt sind. Im Zuge von Voranalysen wurde von einer Vielzahl von Personen, welche in die grundlegenden Hauttypen nach Fitzpatrick eingeteilt wurden, das Reflexionsverhalten der Haut bezüglich der ersten und zweiten Wellenlänge ermittelt. Diese Einteilung in Hauttypen kann nicht streng abgrenzbar sein, da einerseits die Haut keine quantisierten Zustände aufweist und andererseits das Reflexionsverhalten innerhalb eines Hauttyps stark vom aktuellen Zustand wie bspw. der Bräunung abhängt. Somit werden die ermittelten Referenzdaten auch eine gewisse Streuung aufweisen - da es sich im Wesentlichen um einen Zufallsprozess handelt, wird die Verteilung eine Gauß-Verteilung sein. Je nach Abstand von der Hautoberfläche und Hauttyp ist nun jeweils ein Wertepaar bzw. Wertefeldpaar hinterlegt, welches für die beiden Wellenlängen einen Mittelwert und eine Schwankungsbreite abgibt. Beim Vergleich kann dadurch schnell und zuverlässig festgestellt werden, ob das erfasste erste und zweite Signal einer solchen hinterlegten Paarung zugeordnet werden kann. Ist eine Zuordnung nicht möglich, ggf. auch nach mehrmaliger Iteration nicht, wurden die erfassten ersten und zweiten Signal mit sehr großer Wahrscheinlichkeit nicht von Haut reflektiert bzw. handelt es sich um einen Manipulationsversuch.

Eine Weiterbildung besteht auch darin, dass die Referenzdaten der Referenzdatensätze Abweichungsbereiche aufweisen. Im Gegensatz zu den zuvor genannten Bereichsangaben betreffend die Schwankungsbreite der Referenzdaten, wird mit dieser Weiterbildung eine Unschärfe betreffend das vollständige Aufliegen des Kontaktstücks realisiert. Beispielsweise kann es beim Aufsetzen des Kontaktstücks auf Haut über knöchernen Strukturen, bspw. im Gesicht, dazu kommen, dass das Kontaktstück nicht vollständig aufliegt, so dass eine geringfügige Menge Laserstrahlung entweichen kann. Aufgrund der optisch/geometrischen Verhältnisse am bzw. im Kontaktstück, ist die austretende Laserstrahlung jedoch derart stark gestreut, dass eine Gesundheitsgefährdung ausgeschlossen werden kann. Die austretende Laserstrahlung ist somit jedenfalls Laserklasse I. Da die Intensität bzw. Amplitude der erfassten ersten und zweiten Wellenlänge stark abstandsabhängig ist, kann somit bspw. über einen hinterlegten Abweichungsbereich, betreffend die Amplitudenverhältnisse eine Aussage über den Abstand vom Auflagepunkt getroffen werden. Beispielsweise kann somit ein Abstand des Kontaktstück von der Hautoberfläche im Bereich von 0 bis 2mm als sicher angesehen werden, ein Bereich von 2 bis 5mm als Warnbereich und alles über 5mm Abstand führt zu einem Nicht-Sicherer-Betriebszustand Signal.

Um das unvermeidlich vorhandene Umgebungsglicht berücksichtigen zu können, weist gemäß einer Weiterbildung die Sicherungsschaltung einen Umgebungslichtsensor auf, welcher mit der Auswerteschaltung verbunden ist, insbesondere ist dieser mit der Vergleichsschaltung verbunden. Eine zuverlässige Erkennung des Aufliegens auf der Haut ist nur dann gewährleistet, wenn die Intensität des Umgebungslichts innerhalb vorgegebener Bereiche liegt. Zu helles Licht würde bspw. die erfassten Signale überstrahlen.

Ebenfalls zur Berücksichtigung des Umgebungslichts ist gemäß einer Weiterbildung vorgesehen, dass die Sicherungsschaltung einen Pulsgenerator aufweist, welcher zur Ansteuerung der Lichtquelle ausgebildet und mit der Vergleichsschaltung verbunden ist. Durch Variation der Intensität des von der Lichtquelle abgegebenen Lichts, kann dieses aus den erfassten Signalen extrahiert werden, um so einen Einfluss des Umgebungslichts eliminieren zu können.

Eine Weiterbildung besteht ferner darin, dass die Sicherungsschaltung einen weiteren Lichtsensor aufweist, welcher in Richtung des Lichtaustrittsabschnitts ausgerichtet angeordnet ist und mit der Auswerteschaltung verbunden ist, insbesondere mit der Vergleichsschaltung. Das Licht der Lichtquelle wird über den Lichtaustrittsabschnitt in Richtung der Haut ausgestrahlt. Solange das Kontaktstück nicht auf der Haut aufliegt bzw. während der Annäherungsphase an die Haut, wird kaum bzw. wenig Licht aufgrund von Reflexion an der Haut zurückgestrahlt. Je näher das Kontaktstück der Haut kommt, desto mehr Licht wird von der Haut reflektiert, nicht nur in Richtung des Lichteintrittsabschnitts und damit zum Fotodetektor, sondern auch zurück in den Lichtaustrittsabschnitt. Dieses Licht wird erfasst, wobei die maximale Reflexion dann gegeben ist, wenn das Kontaktstück auf der Hautoberfläche aufliegt. Sobald das Kontaktstück abgehoben oder gekippt wird, wird Licht in die Umgebung entweichen und somit die Intensität des vom weiteren Lichtsensor erfassten Signals abfallen. Das vom weiteren Lichtsensor bereitgestellte Signal kann nun als zusätzliches Sicherungskriterium verwendet werden, um das Aufliegen auf der Haut und damit den sicheren Betriebszustand erkennen zu können.

Die Erfindung wird auch durch ein Lasergerät der Laserklasse 1 gelöst, welches eine Laserquelle mit einer Ansteuerschaltung und einer Hautkontakt-Erkennungsvorrichtung umfasst und wobei die Ansteuerschaltung die Laserquelle wird nur bei einem Sicherer-Betriebszustand Signal aktiviert. Die Laserquelle gibt dabei eine Strahlungsleistung größer Laserklasse 1 ab, insbesondere Klasse 4. Durch die anspruchsgemäße Ausbildung ist sichergestellt, dass eine gesundgefährdende Laserquelle derart ausgebildet ist, dass im Betrieb keine gesundheitsgefährdende Strahlung austreten kann und das Lasergerät somit Laserklasse 1 besitzt.

Zum besseren Verständnis der Erfindung wird diese anhand der nachfolgenden Figuren näher erläutert.

Es zeigen jeweils in stark schematisch vereinfachter Darstellung:
- Fig. 1: Ein Prinzipbild der gegenständlichen Hautkontakt-Erkennungsvorrichtung;
- Fig. 2: a) und b) ein Handstück einer optisch wirkenden Energiequelle mit der gegenständlichen Hautkontakt-Erkennungsvorrichtung;
- Fig. 3: a) und b) die Strahlungsverhältnisse beim Annähern und Aufsetzten der gegenständlichen Hautkontakt-Erkennungsvorrichtung auf biologischem Gewebe;
- Fig. 4: Einen schematischen Verlauf der erfassten Intensitäten der beiden Wellenlängen;
- Fig. 5: Eine weitere mögliche Ausführungsform der gegenständlichen Hautkontakt-Erkennungsvorrichtung mit einer Differenzdruckvorrichtung;
- Fig. 6: Eine weitere mögliche Ausführungsform der gegenständlichen Hautkontakt-Erkennungsvorrichtung mit einer Kontaktelektrode;
- Fig. 7: Eine beispielhafte Signalverteilung bei Annäherung an die Haut und Aufsetzen darauf;
- Fig. 8: Einen beispielhaften Signalverlauf zur Verteilung aus Fig. 7.

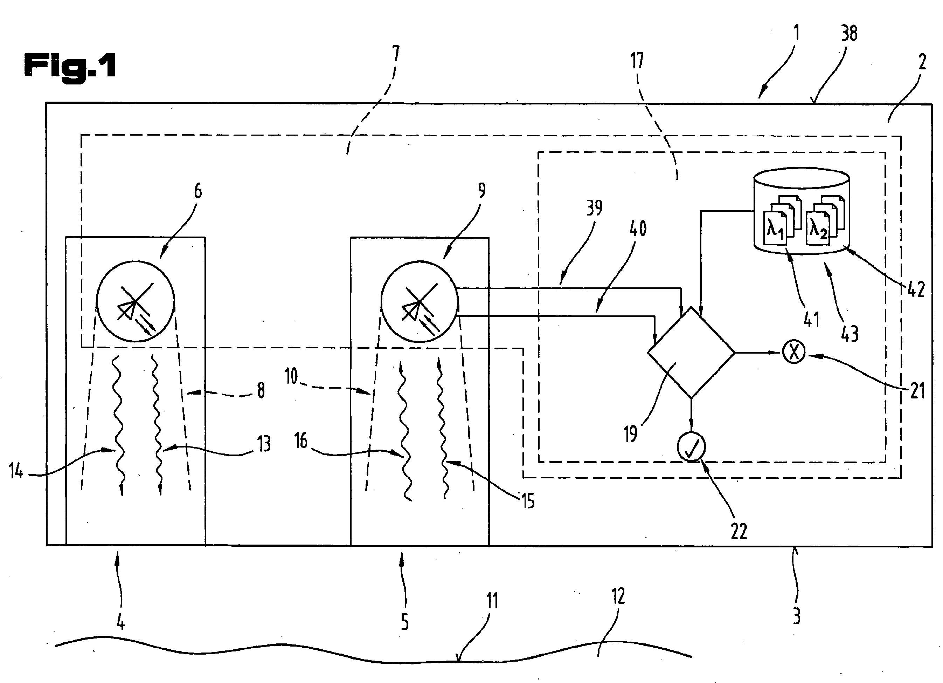

Fig. 1 zeigt eine schematische Darstellung der gegenständlichen Hautkontakt-Erkennungsvorrichtung 1, umfassend ein Kontaktstück 2 mit einer Auflagefläche 3. Die Auflagefläche 3 weist einen Lichtaustritts- 4 und einen Lichteintrittsabschnitt 5 auf. Eine Lichtquelle 6 der Sicherungsschaltung 7 ist mit ihrer Hauptstrahlrichtung 8 der abgegebenen elektromagnetischen Strahlung in Richtung des Lichtaustrittsabschnitts 4 angeordnet. Des Weiteren ist ein Fotodetektor 9 der Sicherungsschaltung 7 mit seinem Erfassungsbereich 10 in Richtung des Lichteintrittsabschnitts 5 angeordnet. Der Lichteintritts- 4 und Lichtaustrittsabschnitt 5 sind voneinander abgegrenzt in der Auflagefläche 3 angeordnet, sodass es keinen direkten Strahlengang der Lichtquelle 6 in Richtung des Fotodetektors 9 gibt. Licht von der Lichtquelle 6 gelangt also nur über eine Reflexion an einer Oberfläche 11 bzw. durch Weiterleitung in einem Gewebe 12 in den Lichteintrittsabschnitt 5 und damit in den Erfassungsbereich 10 des Fotodetektors 9.

Der Kontaktfläche 3 gegenüberliegend weist das Kontaktstück 2 eine Anschlussfläche 38 auf, über welche das Kontaktstück 2 mit dem zu sichernden Gerät 24 verbunden werden kann. Die Anschlussfläche 38 ist bevorzugt derart ausgebildet, dass ein einfaches und sicheres Anordnen des Kontaktstücks 2 am Gerät 24 möglich ist. Bevorzugt wird eine lösbare Anordnung, da nach einer möglichen Ausführungsform, das Kontaktstück 2 ein Kontakt mit biologischem Gewebe haben kann und damit eine entsprechende Reinigung und Sterilisation möglich sein muss. Eine solche Reinigung würde das Gerät 24 wahrscheinlich beschädigen bzw. die Lebensdauer reduzieren, daher ist es von Vorteil, wenn nur das direkt in Gewebekontakt stehende Teil intensiv gereinigt wird. Die Anschlussfläche wird bevorzugt einen Teil einer Ausricht- und Verriegelungsvorrichtung aufweisen, welcher in den, am Gerät 24 gegenüberliegenden, Teil eingreift und das Kontaktstück 2 zuverlässig am zu sichernden Gerät 24 anordnet.

Die Lichtquelle 6 ist dazu ausgebildet, elektromagnetische Strahlung mit zumindest zwei Wellenlängen im optischen Bereich abzugeben, insbesondere eine kurzwellige, erste ausgesandte Wellenlänge 13 und eine gegenüber der ersten ausgesandten Wellenlänge länger wellige, zweite ausgesandte Wellenlänge 14. Der Fotodetektor 9 ist hinsichtlich seiner Sensitivität auf die von der Lichtquelle 6 ausgesandten, elektromagnetischen Strahlung mit der ersten 13 und zweiten Wellenlänge 14 abgestimmt, die elektromagnetische Strahlung erreicht den Fotodetektor 9 als erste empfangene Wellenlänge 15 und zweite empfangene Wellenlänge 16.

Vom Fotodetektor 9 wird die empfangene erste 15 und zweite 16 Wellenlänge jeweils in ein, der Intensität proportionales Signal umgewandelt 39, 40 und in einer Auswerteschaltung 17 analysiert. Insbesondere werden die beiden erfassten Signale 39, 40 einer Vergleichsschaltung 19 mit in einer Datenbank 43 hinterlegten Referenzdatensätzen für die erste 41 und zweite 42 erfasste Signale verglichen. Abhängig vom Vergleich wird entweder ein Nicht-Sicherer-Betriebszustand Signal 21 oder ein Sicherer-Betriebszüstand 22 abgegeben. In den Referenzdatensätzen 41, 42 sind Referenzdaten für jedes der beiden erfassten Signale 39, 40 hinterlegt. Insbesondere wurden diese Referenzdatensätze in Messreihen ermittelt und in der Datenbank 43 hinterlegt. In diesen Messreihen wurden Referenzdaten für unterschiedliche Hauttypen ermittelt, wobei der Absolutbetrag der Intensität der empfangenen Wellenlängen 15, 16 und damit auch die Intensität der erfassten Signale 39, 40, stark vom Hauttyp abhängt. Durch die Verwendung einer Datenbank 43 mit hinterlegten Referenzdatensätzen 41 ,42 ist es möglich, dass eine zuverlässige Annäherung an die Haut bzw. ein Aufliegen auf der Haut erkannt wird, ohne dass ein vorheriger Kalibrierungsschritt erforderlich ist. Der Stand der Technik, beispielsweise die US 7,413,567 B2, offenbart, dass vor Durchführung der eigentlichen Auflageprüfung, die Vorrichtung bewusst auf das Gewebe aufgelegt werden muss, um einen Referenzpunkt ermitteln zu können. Durch diesen Kalibrierungsschritt ist jedoch eine ganz einfache Manipulation möglich, da somit die Vorrichtung auf eine beliebige Unterlage aufgelegt werden kann und somit ein völlig falscher Referenzpunkt ermittelt wird. Im Hinblick auf die Sicherung einer optischen Hochenergie-Strahlungsquelle ist eine derart einfache Manipulationsmöglichkeit jedenfalls von entscheidendem Nachteil.

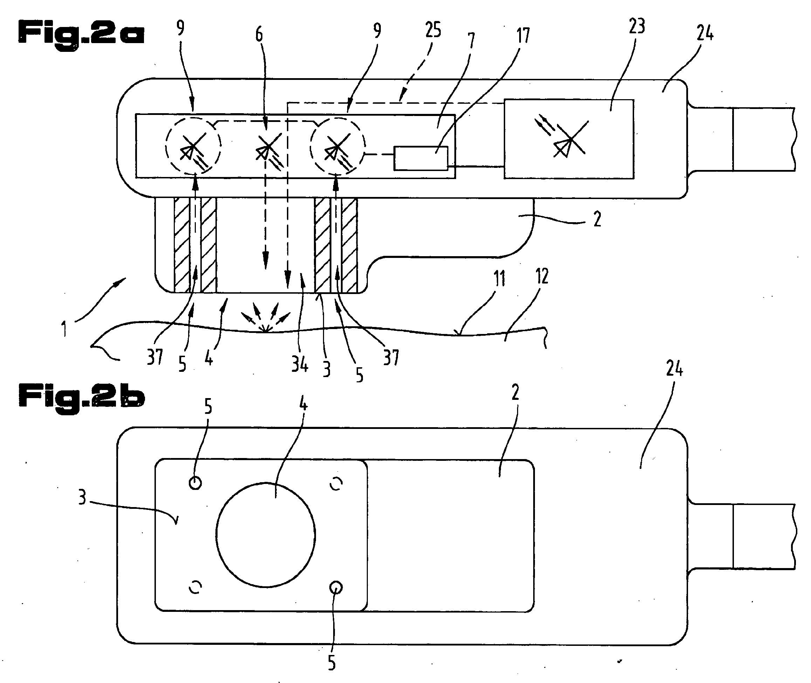

Fig. 2a zeigt eine Darstellung der gegenständlichen Hautkontakt-Erkennungsvorrichtung 1 zur Sicherung einer optischen Hochenergie-Strahlungsquelle 23, welche in einem Handstück 24 angeordnet ist. In einem bevorzugten Ausführungsfall handelt es sich bei der optischen Hochenergie-Strahlungsquelle 23 um einen Laser, insbesondere um einen seitgepumpten Festkörperlaser, welcher optische Hochenergiestrahlung 25 in Richtung einer zu behandelnden Oberfläche 11 abgibt. Bevorzugt erfolgt die Abgabe der Hochenergiestrahlung über den Lichtaustrittsabschnitt 4. Der Energiegehalt der abgegebenen Strahlung 25 ist dabei so hoch, dass beim Eintreffen des Strahls auf das menschliche Auge, eine sofortige Schädigung auftreten würde, insbesondere wird die Strahlung der Klasse 4 nach EN 60 825-1 zugeordnet. Beim Betrieb eines derartigen Gerätes 24 sind ohne die gegenständliche Hautkontakt- Erkennungsvorrichtung jedenfalls umfassende Schutzmaßnahmen erforderlich, insbesondere müssen alle beteiligten Personen im Umgebungsbereich des Geräts 24 zumindest Schutzbrillen tragen. Soll jedoch ein derartig aufwändiger Schutz vermieden werden, muss sichergestellt werden, dass beim Betrieb der optischen Hochenergie-Strahlungsquelle 23 maximal optische Strahlungsenergie in die Umgebung des Geräts austreten kann, welche der Klasse 1 zugeordnet wird und damit als nicht gesundheitsgefährdend eingestuft ist. Mit der gegenständlichen Hautkontakt-Erkennungsvorrichtung 1 wird insbesondere mittels der Auswerteschaltung 17 sichergestellt, dass die Strahlungsquelle 23 nur dann in Betrieb genommen werden kann, wenn von der Sicherungsschaltung 7 bzw. der Auswerteschaltung 17 ein Aufliegen des Kontaktstücks 2 auf der zu behandelnden Oberfläche 11 eindeutig und zuverlässig gewährleistet werden kann. Gemäß vorteilhafter Weiterbildungen ist ferner vorgesehen, dass eine Inbetriebnahme bzw. eine Betriebsaufrechterhaltung auch dann möglich ist, wenn sich das Kontaktstück in einer unmittelbaren Nähe zur Hautoberfläche befindet, als zulässiger Bereich ist bspw. ein Abstand bis zu 5mm vorgesehen.

Die Lichtquelle 6 gibt ihr Licht in Richtung des Lichtaustrittsabschnitts 4 ab. Ohne Kontakt des Kontaktstücks 2 mit der Oberfläche 11 wird das abgegebene Licht auf der Oberfläche 11 auftreffen und zumeist diffus gestreut bzw. zurückreflektiert und gelangt somit über den Lichteintrittsabschnitt 5 zum Fotodetektor 9. Solange das Kontaktstück 2 die Oberfläche 11 nicht berührt, werden beide von der Lichtquelle 6 ausgesandten Wellenlängen vom Fotodetektor erfasst und von der Auswerteschaltung 17 entsprechend analysiert.

Fig. 2b zeigt eine Draufsicht auf die Auflagefläche 3 des Kontaktstücks 2. In der bevorzugten Ausbildung wird der Lichtaustrittsabschnitt 4 einen im Wesentlichen kreisrunden Querschnitt aufweisen und auch im Hinblick auf die Austrittsfläche deutlich größer sein, als der Lichteintrittsabschnitt 5. In der dargestellten, bevorzugten Ausbildung sind zwei Lichteintrittsabschnitte 5 vorgesehen, wobei die beiden Lichteintrittsabschnitte 5 einander um ein Zentrum des Lichtaustrittsabschnitts 4 gegenüberliegend angeordnet sind. In einer weiteren, möglichen Ausbildung könnten gegebenenfalls weitere Lichteintrittsabschnitte vorgesehen sein, wie dies in Fig. 2b durch strichlierte Kreise angedeutet ist. Damit wird eine den Lichtaustrittsabschnitt 4 umschließende Anordnung von Lichteintrittsabschnitten gebildet, sodass auch ein Kippen des Kontaktstückes 2 und damit ein leichtes, seitliches Anheben des Lichtaustrittsabschnittes 4, erkannt werden kann.

In der dargestellten Ausführungsform ist die Sicherungsschaltung 7 in die zu sichernde Vorrichtung 24 integriert, sodass das Kontaktstück im Wesentlichen als Distanzstück dient. Diese Ausführung hat insbesondere den Vorteil, dass das Kontaktstück 2 als Einwegteil ausgebildet sein kann. Gerade bei einem Einsatz im medizinischen bzw. kosmetischen Bereich sind sterile Arbeitsgeräte gefordert, um eine potentiell gesundheitsgefährdende Interaktion eines nicht sterilen Kontaktstücks 2 mit dem behandelten Abschnitt der Oberfläche 11 bzw. des Gewebes 12 zu verhindern. Das Kontaktstück 2 wird dabei mittels einer nicht dargestellten Montage- bzw. Verriegelungsvorrichtung am Handstück 24 angebracht, die Behandlung durchgeführt, und das Kontaktstück 2 anschließend entsorgt. Das Gerät 24 kann für Sterilisations- bzw. Reinigungsmittel dicht ausgeführt sein, beispielsweise kann der Bereich in dem die abgegebenen Lichtstrahlen der gegenständlichen Sicherungsvorrichtung und der Behandlungsstrahl austreten bzw. wie die reflektieren bzw. gestreuten Wellenlängen eintreten, mit einem für diese Wellenlängen transparenten Glas dicht verschlossen sein, sodass eine Reinigung des Geräts bzw. dieser Fläche auch mit aggressiven Reinigungsmitteln möglich ist.

In der Figur nicht dargestellt ist ein weiterer Lichtsensor, der mit seiner Haupterfassungsrichtung in Richtung des Lichtaustrittsabschnitts ausgerichtet angeordnet ist. Von diesem Lichtsensor wird das gesamte in den Lichtaustrittabschnitt zurückreflektierte Licht erfasst, um darüber eine zusätzliche Aussage über die Qualität des Aufliegens des Kontaktstücks auf der Haut ableiten zu können. Insbesondere lässt sich damit auch ein geringfügiges Abheben vom vollständigen Kontakt erkennen.

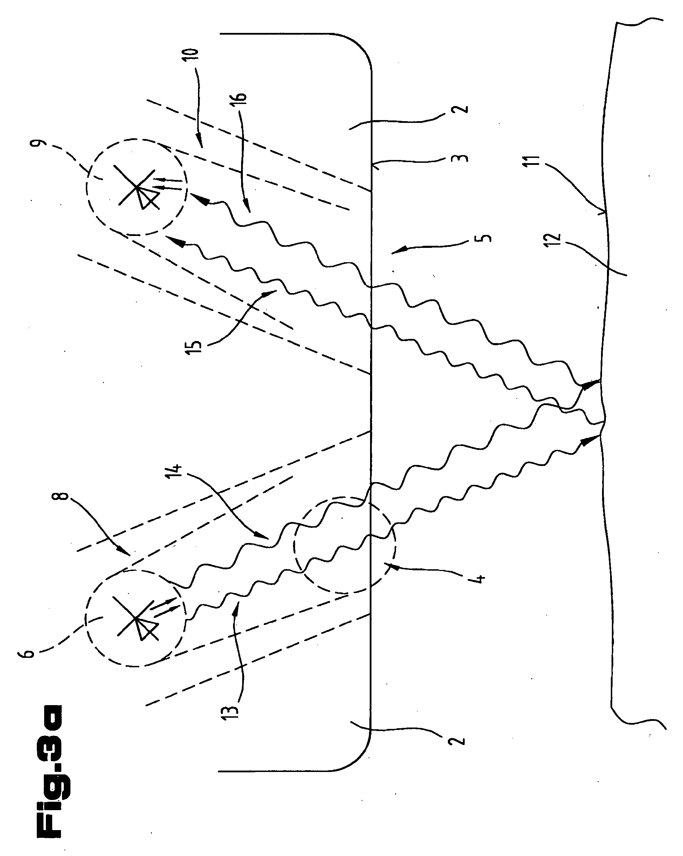

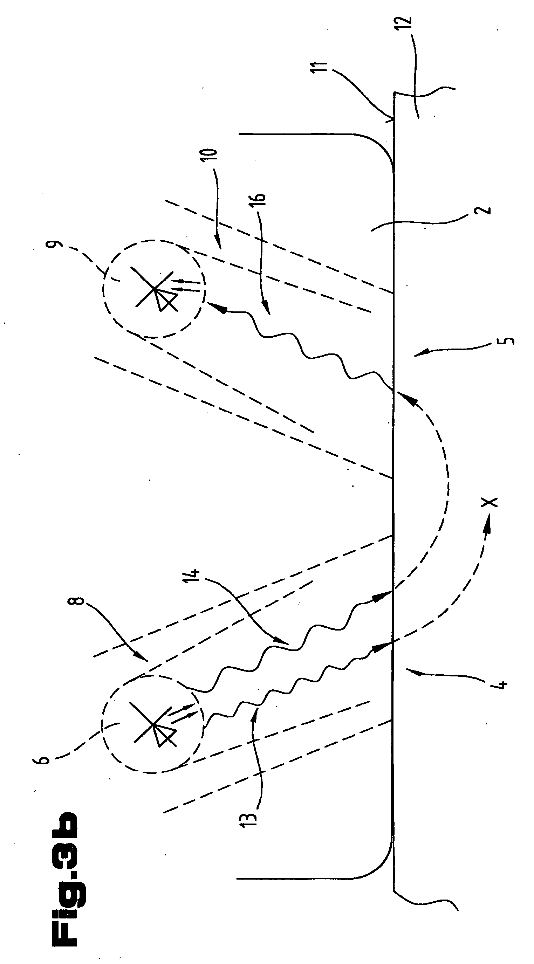

Fig. 3 a) und b) zeigen das Prinzip der Bestimmung des Hautkontaktes mit der gegenständlichen Hautkontakt-Erkennungsvorrichtung 1. Eine Lichtquelle 6 ist mit ihrer Hauptstrahlrichtung 8 in Richtung des Lichtaustrittsabschnitts 4 angeordnet und gibt Licht einer ersten 13 und zweiten 14 Wellenlänge ab. Solange das Kontaktstück 2, insbesondere die Auflagefläche 3, nicht auf der Oberfläche 11 bzw. am Gewebe 12 aufliegt, tritt das von der Lichtquelle 6 abgegebene Licht aus dem Lichtaustrittsabschnitt 4 aus und wird von der Oberfläche 11 bzw. vom Gewebe 12 reflektiert bzw. gestreut und gelangt somit in Richtung des Lichteintrittsabschnitts 5. Das zurückreflektierte bzw. gestreute Licht erreicht den Fotodetektor 9 als erste 15 und zweite 16 Wellenlänge und wird von diesem in ein, der jeweiligen Intensität proportionales erstes 39 und zweites 40 erfasstes Signal umgewandelt.

Die erste 13 und zweite 14 ausgesandte Wellenlänge sind so gewählt, dass für eine Freiraumausbreitung in Luft und für die Reflexion bzw. Streuung an der Oberfläche 11 bzw. am Gewebe 12 die Dämpfungsverhältnisse derart gestaltet sind, dass für beide Wellenlängen ein geringer Dämpfungsbetrag gegeben ist, sodass eine ausreichend hohe Intensität der empfangenen Wellenlängen gewährleistet ist. Das Annähern des Kontaktstückes 2 an die Oberfläche 11 bzw. das Gewebe wird vom Fotodetektor 9 bzw. der Auswerteschaltung dadurch erkannt, dass
- Keine oder nur eine sehr geringe Intensität beider Wellenlängen erfasst wird. In diesem Fall ist das Kontaktstück 2 soweit von der Oberfläche bzw. dem Gewebe entfernt, dass die Intensität der reflektierten bzw. gestreuten Wellenlänge im Bereich der Erfassungsschwelle des Fotodetektors liegt;
- Ein gleichmäßiger Anstieg der Intensitätswerte beider Wellenlängen ermittelt wird. Bei einem derartigen Verlauf nähert sich das Kontaktstück 2 der Oberfläche 11 bzw. dem Gewebe, sodass immer mehr Anteile der an der Oberfläche 11 bzw. am Gewebe gestreuten bzw. reflektierten Wellenlängen in den Lichteintrittsabschnitt 5 gelangen und somit vom Fotodetektor als Intensitätsanstieg erfasst wird.

Fig. 3b zeigt die Verhältnisse beim Aufsetzten des Kontaktstücks 2 auf der Oberfläche 11 bzw. am Gewebe 12. Aufgrund der Ausbreitungsbedingungen für die erste 13 und zweite 14 ausgesandte Wellenlänge im Gewebe 12, wird die zweite ausgesandte Wellenlänge 14 im Gewebe weitergleitet. Die erste ausgesandte Wellenlänge 13 wird vom Gewebe so stark gedämpft wird, dass bereits nach einer sehr geringen Eindringtiefe keine Intensität mehr feststellbar ist. Dies wird dadurch erreicht, dass die erste ausgesandte Wellenlänge 13 in einem Bereich gewählt wird, für den das Gewebe 12 einen hohen Dämpfungsfaktor aufweist, dies entspricht im bevorzugten Ausführungsfall einer Wellenlänge im Bereich kleiner 470nm, insbesondere 450nm. Im Gegensatz dazu ist die zweite ausgesandte Wellenlänge 14 in einem Bereich von 610nm bis 660nm, bevorzugt 635nm, wobei in diesem Frequenzbereich eine vergleichsweise geringere Dämpfung gegeben ist, sodass das eintreffende Licht weitergleitet wird und damit in den Lichteintrittsabschnitt 5 und somit in den Erfassungsbereich 10 des Fotodetektors 9 gelangen kann.

Ein korrektes Aufliegen des Kontaktstückes 2 auf der Oberfläche 11 kann also dadurch erkannt werden, dass vom Fotodetektor 9 nur die zweite Wellenlänge 16 empfangen wird bzw. das im Gegensatz zu den Intensitätsverhältnissen vor dem Aufsetzten des Kontaktstücks 2 auf der Oberfläche 11, ein deutlicher Unterschied der empfangenen Intensität zwischen der ersten 15 und der zweiten 16 empfangenen Wellenlänge besteht. Umgekehrt kann ein geringfügiges Abheben des Kontaktstücks 2 dadurch erkannt werden, dass plötzlich wieder eine erste Wellenlänge 15 empfangen wird, bzw. dass sich ein Unterschied der empfangenen Intensitäten zwischen der ersten 15 und zweiten 16 empfangenen Wellenlänge ausbilden wird. Sobald ein nicht zuverlässiges Aufliegen des Kontaktstücks 2 auf der Oberfläche erkannt wird, kann von der Auswerteschaltung ein Nicht-Sicherer-Betriebszustand Signal abgegeben werden, woraufhin das zu sichernde Gerät, beispielsweise ein Laser der Klasse 4, sofort deaktiviert wird und damit eine Gesundheitsgefährdung anwesender Personen verhindert wird.

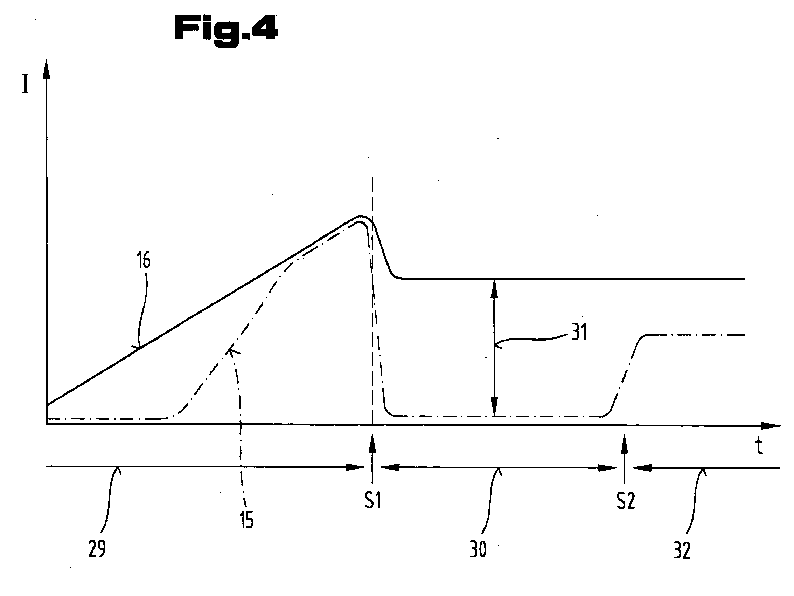

Fig. 4 zeigt schematisch den Intensitätsverlauf der empfangenen Wellenlängen beim Annähern an die Oberfläche bis zum Aufsetzen und ein danach folgendes partielles Lösen des Kontakts, wodurch ein potentiell gesundheitsgefährdender Betriebszustand gegeben ist, was in jedem Fall verhindert werden muss. Zur Vereinfachung der Darstellung ist der Intensitätsverlauf der beiden Wellenlängen durch eine unterschiedliche Strichart dargestellt. Der dargestellte Intensitätsverlauf zeigt einen möglichen, beispielhaften Intensitätsverlauf, insbesondere soll das Prinzip dargestellt werden, ein tatsächlicher Intensitätsverlauf kann davon jedenfalls abweichen. Zur Vereinfachung der Darstellung wurden die Intensitätswerte der beiden empfangenen Wellenlängen einander angeglichen, die absoluten Intensitätswerte werden sich ggf. deutlich unterscheiden. Bei der dargestellten Situation kommt es auf den Verlauf an, daher sind die Absolutwerte zweitrangig. Auch ist hier eine Situation dargestellt, bei der ein vollständiges Aufliegen sichergestellt werden muss. Gemäß vorteilhafter Weiterbildungen kann auch ein geringfügiges Abheben als sicherer Betriebszustand toleriert werden.

Während einer Annäherungsphase 29 wird die Intensität der ersten 15 und zweiten 16 empfangenen Wellenlänge ansteigen, da aufgrund der Annäherung des Kontaktstücks an die Oberfläche, mehr Licht vom Lichtaustrittsabschnitt durch Reflexion bzw. Streuung an der Oberfläche in den Lichteintrittsabschnitt gelangt. Die zweite Wellenlänge wird gut von der Haut reflektiert, so dass bereits in einem größeren Abstand die zweite Wellenlänge 16 empfangen wird. Die erste Wellenlänge wird deutlich schwächer von der Haut reflektiert, so dass es erst bei einem deutlich geringeren Abstand zu einer Reflexion und damit zu einer zweiten empfangenen Wellenlänge 15 kommen wird. Mit zunehmender Annäherung an die Haut werden die Intensitäten der beiden empfangenen Wellenlängen 15, 16 ansteigen, wesentlich dabei ist, dass beide Intensitäten ansteigen müssen. Im Diagramm ist dies durch ein betragsmäßig gleiches Ansteigen der Intensitätskurven dargestellt.

Beim Aufsetzten des Kontaktstücks auf die Oberfläche werden nun beide Wellenlängen über den Lichtaustrittsabschnitt in das Gewebe eingeleitet und breiten sich darin entsprechend der Ausbreitungsbedingungen aus. Maßgeblich ist hierbei die Dämpfung, welche eine Wellenlänge durch das Gewebe erfährt. Der Zeitpunkt des Aufsetzens ist im Diagramm durch den Abschnitt S1 gekennzeichnet. Für die erste empfangene Wellenlänge 15 kommt es aufgrund der starken Dämpfung durch das Gewebe zu einem starken Abfall der erfassten Intensität, bis hin zu einer völligen Auslöschung, sodass vom Fotodetektor für die erste Wellenlänge 15 keine, oder nur eine sehr geringe Intensität erfassbar ist. Da auch die zweite Wellenlänge 16 der Dämpfung durch das Gewebe unterliegt, wird auch hier die erfasste Intensität zurückgehen, allerdings in einem viel geringeren Umfang, als dies für die Intensität der ersten Wellenlänge 15 der Fall ist. Ein sicherer Betriebszustand 30 ist also dadurch gekennzeichnet, dass eine große Differenz 31 zwischen den ermittelten Intensitäten der zweiten 16 und ersten 15 empfangenen Wellenlänge besteht, bzw. dass die Intensität der ersten Wellenlänge 15 völlig verschwindet. In der Darstellung besteht in der Annäherungsphase 29 kurz vor dem Kontakt, keine Differenz zwischen den Intensitäten der beiden Wellenlängen. Die ist nur aus Darstellungsgründen so gewählt. Wie bereits erwähnt, können die absoluten Intensitätswerte auch hier bereits eine Differenz aufweisen, diese wird sich jedoch beim Aufsetzen auf der Oberfläche deutlich vergrößern.

Wird das Kontaktstück nun ganz leicht abgehoben, kann wieder Licht vom Lichtaustrittsabschnitt direkt in den Lichteintrittsabschnitt gelangen und es wird wiederum die erste Wellenlänge 15 mit einer merkbaren Intensität erfasst, wodurch sich auch der Differenzbetrag zwischen der ersten 15 und zweiten 16 empfangenen Wellenlänge ändert. Dies ist im Diagramm ab dem Zustand S2 der Fall, was somit eindeutig einen unsicheren Betriebszustand 32 charakterisiert. Vom Auswertemodul wird dann das Nicht-Sicherer-Betriebszustands Signal abgegeben und die zu sichernde Vorrichtung kann somit deaktiviert werden, um eine Gesundheitsgefährdung beteiligter Personen zu verhindern.

Wesentlich für die zuverlässige Erkennung einer Annäherung der Vorrichtung an Hautgewebe und abschließendes Aufsetzen ist der Verlauf der erfassten Intensitäten. Diesbezüglich ist gemäß einer Weiterbildung vorgesehen, dass die Vergleichsschaltung zustandsabhängig ausgebildet ist, insbesondere als state-machine. Eine state-machine ermöglich bspw. die Prüfung, ob sich eine Eingangsgröße gemäß einem vorgegebenen Verlauf verhält, ohne für den Verlauf alle Möglichkeiten im Detail spezifizieren zu müssen. Eine Annäherung mit anschließendem Aufsetzen kann von einer state-machine folgendermaßen geprüft werden.
- Die Intensität der zweiten empfangenen Wellenlänge steigt; die Intensität der ersten empfangenen Wellenlänge ist unter einer Erfassungsschwelle.
- Es wird eine erste Wellenlänge erfasst, deren Intensität steigen muss, die Intensität der erfassten zweiten Wellenlänge muss weiterhin steigen.
- Nach Erreichen eines Maximums für beide Wellenlängen, fallen die Intensitäten beider erfasster Wellenlängen schnell und stark ab, die Intensität der ersten erfassten Wellenlänge verschwindet bzw. wird sehr gering.
   Mit diesem Zustand wird ein Aufsetzten bzw. Aufliegen der Vorrichtung auf der Haut erkannt.
- Ein Aufliegen ist nun solange gegeben, bis wieder eine erste Wellenlänge erfasst wird, oder bis eine überproportional große Intensitätsschwankung der zweiten Wellenlänge erfasst wird.

Die dargestellten Schritte sind nur beispielhaft zu sehen, um das Prinzip zu veranschaulichen. Insbesondere können weitere Schritte vorgesehen sein, um Zwischenzustände realisieren zu können, bspw. ein Erkennen eines Nahbereichs der Hautoberfläche.

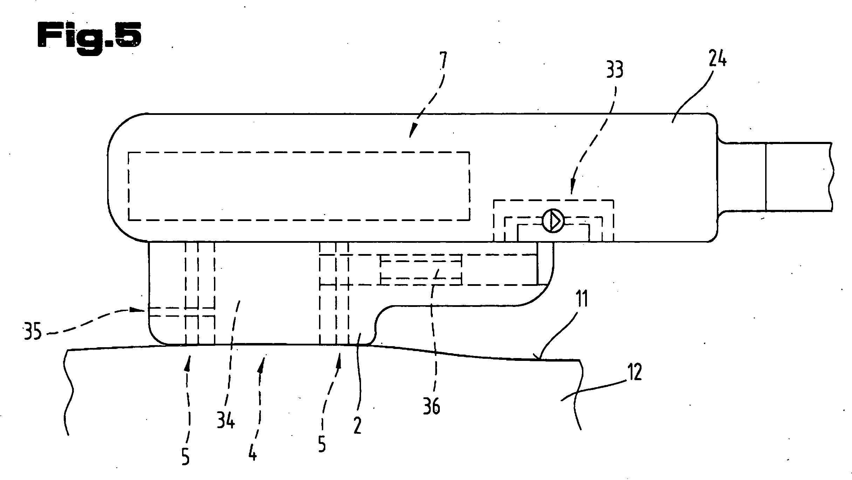

In Fig. 5 ist eine weitere mögliche Ausführungsform dargestellt, bei der der Durchbruch 34 des Lichtaustrittsabschnitts 4 des Kontaktstücks 2 mit einer Vorrichtung zur Erzeugung eines Differenzdrucks 33 verbunden ist. Beim Aufliegen des Kontaktstücks 2 auf der Oberfläche ist der Durchbruch 34 des Lichtaustrittsabschnitts 4 im Kontaktstück 2 auf der einen Seite durch die Hautoberfläche 11 und auf der gegenüberliegenden Seite durch das Handstück 24, an dem das Kontaktstück angeordnet ist, weitestgehend dicht abgeschlossen. Von der Differenzdruckvorrichtung 33 kann daher im Durchbruch 34 ein gegenüber der Umgebung reduzierter Luftdruck ausgebildet werden, wobei aufgrund des weitestgehend dichten Abschluss, dieser Differenzdruck im Wesentlichen bestehen bleiben. Es ist jedoch auch möglich, dass im Bereich des Durchbruchs 34 ein definierter Luftstrom ausgebildet wird, damit Reaktionsprodukte, welche beim Einwirken der im Handstück 24 angeordneten, optischen Hochenergie-Strahlungsquelle auf die zu bearbeitende Oberfläche entstehen, aus dem Behandlungsgebiet abtransportiert werden. Dazu weist das Kontaktstück 2 bevorzugt eine definierte Zuführvorrichtung 35 für Zuluft auf. Wird von der Differenzdruckvorrichtung 33 der Durchbruch 34 mit einem Unterdruck beaufschlagt, strömt über die Luftzuführöffnung 35 eine definierte Menge Luft in den Durchbruch 34, durchströmt diesen, sodass die Abtragsprodukte aus dem Durchbruch abtransportiert und beispielsweise in einem Filter 36, welcher im Abluftkanal angeordnet ist, gesammelt werden. Zur Auswerten des Verlaufs des Differenzdrucks weist das Auswertemodul eine Differenzdruckerfassungsvorrichtung auf, mittels der erkannt werden kann, wenn das Kontaktstück 2 leicht von der Oberfläche 11 abgehoben wird. Bei einem Anheben wird sich der von der Differenzdruckvorrichtung 33 hergestellte Druckunterschied im Durchbruch verändern. Beispielsweise ist es auch möglich, dass das Aufsetzten des Kontaktstücks 2 auf der Hautoberfläche 11 mittels der zuvor beschriebenen optisch wirkenden Vorrichtung ermittelt wird, danach die Differenzdruckvorrichtung 33 aktiviert wird und ein Differenzdruck im Durchbruch 34 ausgebildet wird. Dieser Differenzdruck wird als Referenzdruck angenommen, und anschließend das Aufliegen des Kontaktstücks 2 auf der Oberfläche durch Überwachung des Differenzdrucks kontrolliert. Ähnlich wie zuvor kann auch für den ermittelten Differenzdruck ein Schwellwert hinterlegt sein, welcher vom Auswertemodul mit dem aktuell ermittelten Differenzwert verglichen wird, um somit eine systematische Manipulation der Differenzdruckmessung zu verhindern.

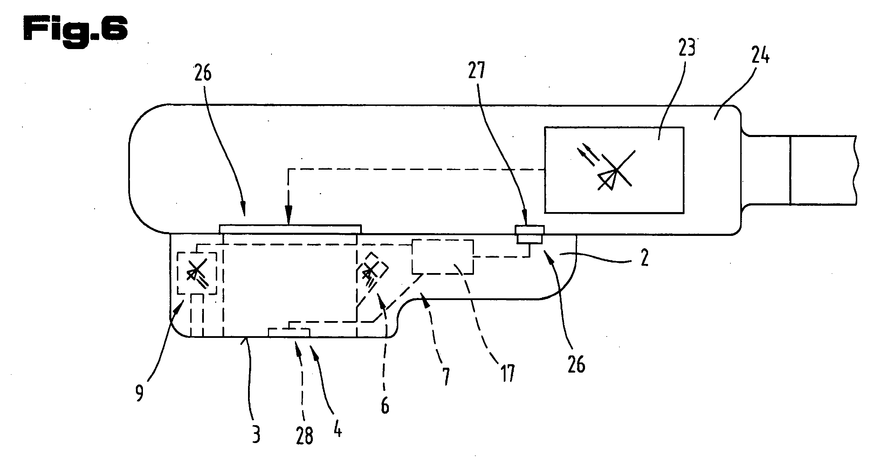

Fig. 6 zeigt eine weitere mögliche Ausführung der gegenständlichen Hautkontakt-Erkennungsvorrichtung, bei der die Sicherungsschaltung 7 im Kontaktstück 2 integriert angeordnet ist. Die optische Hochenergie-Strahlungsquelle 23 wird ihre Strahlung 25 über ein Fenster 26 im Gehäuse des Handstücks 24 nach außen abgeben, insbesondere in den Lichtaustrittsabschnitt 4 des Kontaktstücks 2. Zur Versorgung der Lichtquelle 6 und der Auswerteschaltung 17 bzw. des Fotodetektors 9, weist das Kontaktstück 2 einen dem zu sichernden Gerät 24 zugewandten Kontaktabschnitt 26 auf, welcher bei der Anordnung des Kontaktstücks 2 am zu sichernden Gerät 24 mit einem gegenüber im Handstück 24 angeordneten Kontaktabschnitt 27 verbunden ist. Die beiden Kontaktabschnitte 26, 27 sind bevorzugt zur Übertragung elektrischer Energie und gegebenenfalls elektrischen Signalen ausgebildet. Gemäß einer Weiterbildung kann an der Auflagefläche 3 noch zumindest eine Kontaktelektrode angeordnet sein, welche mit der Auswerteschaltung 17 verbunden ist. Dadurch ist eine Messung elektrischer Kenngrößen der Oberfläche bzw. des Gewebes, auf welchem das Kontaktstück 2 aufliegt, möglich. Die Ermittlung elektrischer Kenngrößen mittels der Kontaktelektrode 28 und der Auswerteschaltung 17 bringt einen zusätzlichen Sicherungsaspekt mit sich, da über die elektrischen Kenngrößen beispielsweise auch auf den Feuchtigkeitsgehalt des Gewebes rückgeschlossen werden kann und somit eine Erhöhung der Zuverlässigkeit des Erkennens des Aufliegens auf der Haut erreicht wird. Mittels der Kontaktelektrode kann beispielsweise ein Widerstandwert oder ein kapazitiver bzw. dielektrischer Verschiebungsstrom bestimmt werden. Diese Weiterbildung ermöglicht eine zusätzliche Steigerung der Erkennungssicherung des Aufliegens des Kontaktstücks 2 auf der Oberfläche. Der Einsatz dieser Weiterbildung bedingt allerdings ein Aufliegen eines elektrischen Kontaktes auf der Haut, so dass hier ggf. gesonderte Sicherheitsvorschriften zur Anwendung kommen, um eine Gefährdung einer Person durch die elektrische Kontaktierung zu verhindern.

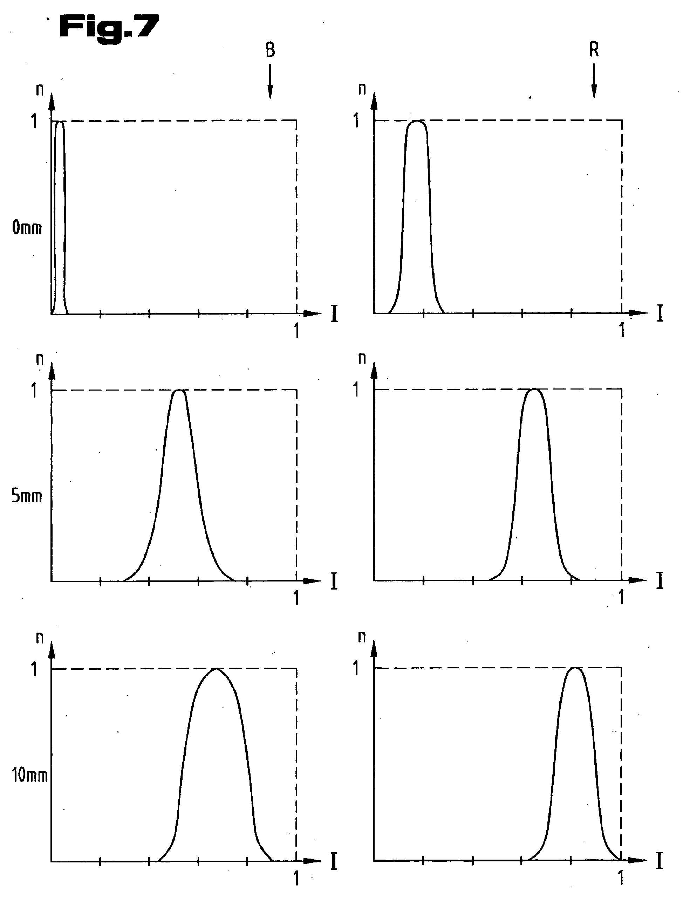

Figur 7 zeigt einen grundsätzlichen Verlauf der als Referenzdatensatz hinterlegten Intensitätsverteilung für einen Hauttyp, in diesem Fall Hauttyp III. Die Referenzdaten für die restlichen Hauttypen sehen grundsätzlich ähnlich aus, es bestehen jedoch deutliche Unterschiede bei der Ausgangssituation beim Maximalwert und bei der Intensitätsverteilung im Auflagefall. Diese Kurven wurden in Versuchsreihen aufgenommen, in dem bei einer Mehrzahl von Personen unterschiedlichen Hauttyps, mit einem Messkopf der Intensitätsverlauf der reflektierten Wellenlängen bei der Annäherung an die Haut ermittelt wurde. Wie bereits zuvor erwähnt, kommt es aufgrund von Unsicherheiten bei der Hauttypzuordnung, aktuellen Schwankungen der Hautbeschaffenheit (insbesondere Bräunung) und sonstige tagesabhängige Faktoren, zu einer Streuung der erfassten Ergebnisse. Da es sich im Wesentlichen um Zufallsprozesse handelt, wird die Verteilungskurve eine Gauß-Verteilung sein. Im Idealfall müsste die Verteilungsfunktion eine schmale Vertikale (Dirac) sein.

Die linke Spalte zeigt das Verhalten für die erste Wellenlänge, blaues bis UV Licht, die rechte Spalte zeigt die zweite Wellenlänge - rotes Licht. Auf der Abszisse ist normiert die erfasste Intensität der reflektierten Wellenlänge aufgetragen, die Ordinate zeigt normiert die Verteilung. Zeile 1 zeigt die Situation beim Aufliegen des Messkopfs auf dem Gewebe, Zeile 2 zeigt die Situation bei einem Abstand von 5mm zwischen Gewebe und Messkopf und Zeile 3 zeigt die Situation bei einem Abstand von 10mm.

Klar zu erkennen ist, dass die erfasste Intensität beider Wellenlängen sinkt, aus Darstellungsgründen ist für Blau, 0mm noch ein Signal dargestellt, tatsächlich ist das Signal sehr schwach, beinahe Null. Auch erkennbar ist, dass die Breite der Verteilungsfunktion bei der Annäherung sinkt. Eine Erklärung könnte sein, dass bei einem größeren Abstand mehr Störanteile den Fotodetektor erreichen können und somit Artefakte produzieren. In der Figur wird von einem Abstand von 10mm ausgegangen, bei dem nach Untersuchungen die maximale Reflexion feststellbar ist.

Eine Annäherung an Hauttyp III ist nun dadurch charakterisiert:
- Dass beim Maximalwert (10mm Abstand) rot stark reflektiert wird, ca. mit dem normierten Faktor 0.8, das tatsächlich erfasste zweite Signal wird in einer geringen Schwankungsbreite um diesen Mittelwert liegen. Blau wird deutlich schwächer reflektiert, der Mittelwert liegt bei ca. 0.6, ferner weist das tatsächlich erfasste erste Signal eine deutlich größere Schwankungsbreite auf.
- Beim Aufliegen verschwindet das erste erfasste Signal (blau) fast vollständig, während für das zweite erfasste Signal (rot) noch ein deutlicher Signalanteil mit einem Mittelwert von ca. 0.15 erfasst wird.

In den Referenzdatensätzen sind nun solche Verläufe hinterlegt, so dass bei der Annäherung geprüft werden kann, ob der erfasste Verlauf mit einem hinterlegten, erwarteten Verlauf übereinstimmt. Durch hinterlegte Bereichsangaben lassen sich somit auch statistische Ausreißer in den Griff bekommen.

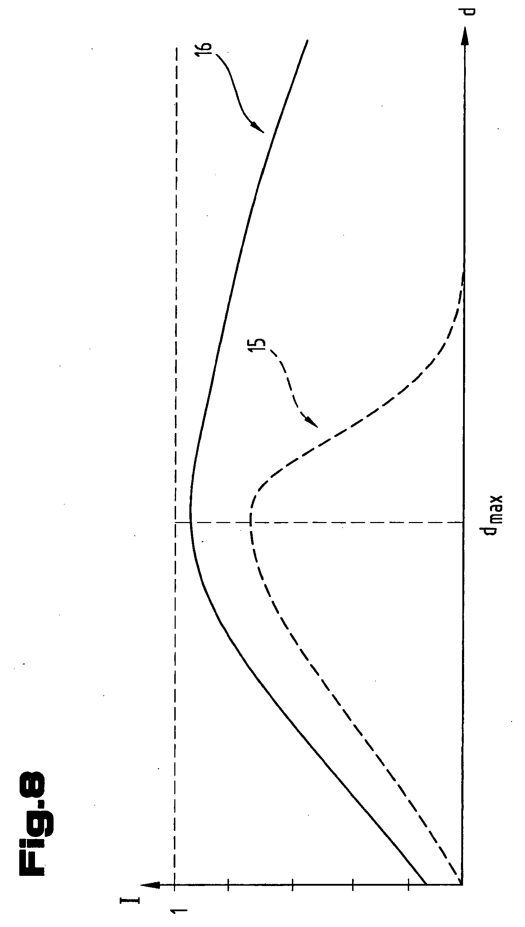

Figur 8 zeit einen zeitlichen Verlauf der Signale aus Figur 7, durchgezogen ist die zweite Wellenlänge 16 (rot), strichliert ist die erste Wellenlänge 15 (blau) dargestellt. Wie bereits erwähnt, gibt es bei Annäherung einen Maximalwert der Reflexion, danach fallen die Intensitäten beider reflektierter Wellenlängen ab. Die Intensitätsverteilung beim Maximum, in der Figur dmax, und die Endintensitäten beim Aufliegen auf die Haut, sind charakteristisch für die Annäherung an Haut und insbesondere lässt sich darüber der Hauttyp charakterisieren. Bei der Annäherung wird zuerst rotes Licht 16 mit steigender Intensität erfasst, wobei bereits bei großem Abstand Signalanteile erfasst werden. Erst bei geringem Abstand wird blaues Licht 15 von der Haut ausreichend reflektiert, um erfasst werden zu können. Nach Unterschreiten des Maximalwerts-Abstands dmax fallen die Intensitäten ab, der Einfachheit halber wurde in der Figur ein im Wesentlichen ein linearer Abfall dargestellt. Obwohl der tatsächliche Verlauf davon abweichen kann, ist aufgrund des bereits geringen Abstands zur Haut, eine lineare Näherung zulässig.

Der wesentliche Vorteil der gegenständlichen Vorrichtung bzw. des gegenständlichen Verfahrens liegt darin, dass durch die Erfassung eines Intensitätsverlauf zweier reflektierter Wellenlängen, eine Annäherung erkannt werden kann, ferner geprüft werden kann, ob die Annäherung an Haut bzw. biologisches Gewebe erfolgt. Zusätzlich kann ein sicheres Aufliegen bzw. die Einhaltung bestimmter Abstandsbereiche ermittelt werden. Im Hinblick auf einen Einsatz im medizinischen Bereich ist es von besonderem Vorteil, dass diese Erfassung kontaktlos durchgeführt werden kann.

Abschließend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Des Weiteren können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten der Hautkontakt-Erkennungsvorrichtung, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

In den Fig.5 und 6 sind weitere und gegebenenfalls für sich eigenständige Ausführungsform der Hautkontakt-Erkennungsvorrichtung gezeigt, wobei wiederum für gleiche Teile gleiche Bezugszeichen bzw. Bauteilbezeichnungen wie in den vorangegangenen Figuren verwendet werden. Um unnötige Wiederholungen zu vermeiden, wird auf die detaillierte Beschreibung in den vorangegangenen Figuren hingewiesen bzw. Bezug genommen.

Der Ordnung halber sei abschließend darauf hingewiesen, dass zum besseren Verständnis des Aufbaus der Hautkontakt-Erkennungsvorrichtung diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Vor allem können die einzelnen in den Fig. 1 bis 8 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

**Bezugszeichenaufstellung**

| | | | |
|---|---|---|---|
| 1 | Hautkontakt-Erkennungsvorrichtung | 31 | Differenz |
| | | 32 | Unsicherer Betriebszustand |
| 2 | Kontaktstück | 33 | Vorrichtung zur Erzeugung eines Differenzdrucks |
| 3 | Auflagefläche | | |
| 4 | Lichtaustrittsabschnitt | 34 | Erster Durchbruch |
| 5 | Lichteintrittsabschnitt | 35 | Luft-Zuführöffnung |
| | | | |
| 6 | Lichtquelle | 36 | Filter |
| 7 | Sicherungsschaltung | 37 | Zweiter Durchbruch |
| 8 | Hauptstrahlrichtung | 38 | Anschlussfläche |
| 9 | Fotodetektor | 39 | Erstes erfasstes Signal |
| 10 | Erfassungsbereich | 40 | Zweites erfasstes Signal |
| | | | |
| 11 | Oberfläche | 41 | Referenzdatensatz für erste erfasste Signale |
| 12 | Gewebe | | |
| 13 | Erste ausgesandte Wellenlänge | 42 | Referenzdatensatz für zweite erfasste Signale |
| 14 | Zweite ausgesandte Wellenlänge | | |
| 15 | Erste empfangene Wellenlänge | 43 | Datenbank |
| | | | |
| 16 | Zweite empfangene Wellenlänge | | |
| 17 | Auswerteschaltung | | |
| 18 | Differenzbildungsschaltung | | |
| 19 | Vergleichsschaltung | | |
| 20 | Grenzwert | | |
| | | | |
| 21 | Nicht-Sicherer-Betriebszustands-Signal | | |
| 22 | Sicherer-Betriebszustands-Signal | | |
| 23 | Optische Hochenergie Strahlungsquelle | | |
| 24 | Handstück, Gerät | | |
| 25 | Optischer Hochenergie Strahl | | |
| | | | |
| 26 | Kontaktabschnitt | | |
| 27 | Kontaktabschnitt | | |
| 28 | Kontaktelektrode | | |
| 29 | Annäherungsphase | | |
| 30 | Sicherer Betriebszustand | | |

## Patentansprüche

1. Verfahren zur Erkennung der Annäherung an und des Aufliegens eines Geräts (24) auf einer Hautoberfläche (11, 12), mit einer Hautkontakt-Erkennungsvorrichtung (1), welche Hautkontakt-Erkennungsvorrichtung (1) ein Kontaktstück (2) mit einer Auflagefläche (3) und eine Sicherungsschaltung (7) umfasst,
wobei die Sicherungsschaltung (7) eine Lichtquelle (6), einen Fotodetektor (9) und eine Auswerteschaltung (17) aufweist, welche Auswerteschaltung (17) mit dem Fotodetektor (9) verbunden ist, wobei
• von der Lichtquelle (6) über einen Lichtaustrittsabschnitt (4) der Auflagefläche (3) eine erste (13) und zweite (14) ausgesandte Wellenlänge in Richtung der Hautoberfläche (11, 12) abgegeben wird;
• die über einen Lichteintrittsabschnitt (5) der Auflagefläche (3) den Fotodetektor (9) erreichende erste (15) und zweite (16) Wellenlänge vom Fotodetektor (9) erfasst wird und in ein, der Intensität proportionales, erstes (39) und zweites (40) erfasstes Signal umgewandelt wird;
• von der Auswerteschaltung (17) für das erste (39) und zweite (40) erfasste Signal ein erster und zweiter zeitlicher Signalverlauf gebildet wird;
• wobei von einer Vergleichsschaltung (19) der Auswerteschaltung (17) der erste und zweite zeitliche Signalverlauf mit den, in einer Datenbank (43) der Auswerteschaltung (17) hinterlegten Referenzdatensätzen (41, 42) verglichen wird;
• wobei ferner von der Auswerteschaltung (17) ein Sicherer-Betriebszustand Signal (22) dann abgegeben wird,
• wenn der erste und zweite erfasste Signalverlauf mit Referenzdaten für erste (41) und zweite (42) erfasste Signalverläufe übereinstimmt.

2. Verfahren nach Anspruch 1, **dadurch gekennzeichnet, dass** der erste und zweite Signalverlauf mittels einer Glättungsfunktion ermittelt wird.

3. Verfahren nach einem der Ansprüche 1 oder 2, **dadurch gekennzeichnet, dass** die Vergleichsschaltung (17) einen unscharfen Vergleich durchführt und neben der Übereinstimmung, zumindest ein zweites Betriebszustands-Signal abgibt, insbesondere ein Warnsignal.

4. Verfahren nach einem der Ansprüche 1 bis 3, **dadurch gekennzeichnet, dass** bei der Bildung der zeitlichen Signalverläufe, eine Maximalwertfindung durchgeführt wird und/oder dass bei der Bildung der zeitlichen Signalverläufe, jeweils eine Trendanalyse durchgeführt wird.

5. Verfahren nach Anspruch 4, **dadurch gekennzeichnet, dass** die ermittelten Trends mit in den Referenzdatensätzen (41, 42) hinterlegten Trends oder mit aus den Referenzdatensätzen ermittelten Referenztrends verglichen werden.

6. Verfahren nach einem der Ansprüche 1 bis 5, **dadurch gekennzeichnet, dass** beim Aufliegen des Geräts (24) bzw. des Kontaktstücks (2) auf der Haut näherungsweise kein erstes Signals (39) erfasst wird.

7. Verfahren nach einem der Ansprüche 4 bis 6, **dadurch gekennzeichnet, dass** die ermittelten Signalverläufe, ab dem ermittelten Maximalwert linear interpoliert werden.

8. Verfahren nach einem der Ansprüche 1 bis 7, **dadurch gekennzeichnet, dass** der Lichteintrittsabschnitt (4) von einer Vorrichtung zur Erzeugung eines Differenzdrucks (33) mit einem Unterdruck oder einem Überdruck beaufschlagt wird und der Differenzdruck von einer Druck-Messvorrichtung überwacht wird, und bei Überschreiten eines Druck-Grenzwertes des Differenzdrucks das Sicherer-Betriebszustand Signal (22) und bei Unterschreiten eines Druck-Grenzwertes des Differenzdrucks das Nicht-Sicherer-Betriebszustand Signal (21) abgegeben wird.

9. Hautkontakt-Erkennungsvorrichtung (1) für ein zu sicherndes Gerät (24), ausgebildet zur Durchführung eines Verfahrens nach einem der Ansprüche 1 bis 8, umfassend
ein Kontaktstück (2) mit einer Auflagefläche (3),
eine Sicherungsschaltung (7) mit einer Lichtquelle (6), einem Fotodetektor (9) und einer Auswerteschaltung (17), welche Auswerteschaltung (17) mit dem Fotodetektor (9) verbunden ist,
wobei die Auflagefläche (3) einen Lichtaustritts- (4) und einen davon abgegrenzten Lichteintrittsabschnitt (5) aufweist,
wobei die Lichtquelle (6) eine Hauptstrahlrichtung (8) der abgegebenen elektromagnetischen Strahlung aufweist,
welche Hauptstrahlrichtung (8) in Richtung des Lichtaustrittsabschnitts (4) ausgerichtet ist,
wobei der Fotodetektor (9) einen Erfassungsbereich (10) für elektromagnetische Strahlung aufweist,
welcher Erfassungsbereich (10) in Richtung des Lichteintrittsabschnitts (5) ausgerichtet ist, wobei die Lichtquelle (6) zur Abgabe von Licht einer ersten (13) und zumindest einer zweiten (14) Wellenlänge ausgebildet ist
und der Fotodetektor (9) für die erste (13) und zweite (14) Wellenlänge sensitiv ist und zur Umwandlung einer empfangenen ersten Wellenlänge (15) in ein erstes erfasstes Signal (39) und der empfangenen zweiten Wellenlänge (16) in ein zweites erfasstes Signal (40) ausgebildet ist,
wobei
die Auswerteschaltung (17) eine Datenbank (43) aufweist, in der Referenzdatensätze (41, 42) mit Referenzdaten für erfasste erste (39) und zweite (40) Signale hinterlegt sind, wobei die Auswerteschaltung dazu ausgebildet ist, aus dem erfassten ersten (39) und zweiten (40) Signal, einen ersten und zweiten zeitlichen Signalverlauf zu bilden,
und dass ferner die Auswerteschaltung (17) eine Vergleichsschaltung (19) aufweist, welche Vergleichsschaltung (19) zum Vergleich des ersten und zweiten zeitlichen Signalverlaufs mit den in der Datenbank (43) hinterlegten Referenzdatensätzen (41, 42) und zur Abgabe eines Sicherer-Betriebszustand Signal (22) ausgebildet ist.

10. Hautkontakt-Erkennungsvorrichtung Anspruch 9, **dadurch gekennzeichnet, dass** die erste Wellenlänge (13) kleiner 470nm ist, insbesondere 450nm und/oder dass die zweite Wellenlänge (14) im Bereich von 610nm bis 660nm, liegt, insbesondere bei 635nm.

11. Hautkontakt-Erkennungsvorrichtung nach einem der Ansprüche 9 oder 10, **dadurch gekennzeichnet, dass** das Kontaktstück (2) eine der Kontaktfläche (3) gegenüber liegende Anschlussfläche (38) aufweist, wobei die Anschlussfläche (38) zur Anordnung des Kontaktstücks (2) an einem zu sichernden Gerät (24) ausgebildet ist.

12. Hautkontakt-Erkennungsvorrichtung nach einem der Ansprüche 9 bis 11, **dadurch gekennzeichnet, dass** der Lichtaustrittsabschnitt (4) über einen ersten Durchbruch (34) im Kontaktstück (2) und/oder dass der Lichteintrittsabschnitt (5) über einen zweiten, als Lichtleiter ausgebildeten Durchbruch (37) im Kontaktstück (2) mit der Anschlussfläche (38) verbunden ist.

13. Hautkontakt-Erkennungsvorrichtung nach einem der Ansprüche 9 bis 12, **dadurch gekennzeichnet, dass** die Lichtquelle (6) durch zumindest eine Leuchtdiode oder einer Laserdiode gebildet ist.

14. Hautkontakt-Erkennungsvorrichtung nach einem der Ansprüche 9 bis 13, **dadurch gekennzeichnet, dass** die Lichtquelle (6) im Lichtaustrittsabschnitt (4) angeordnet ist.

15. Hautkontakt-Erkennungsvorrichtung nach einem der Ansprüche 9 bis 14, **dadurch gekennzeichnet, dass** in der Auflagefläche (3) zumindest zwei Kontaktelektroden (28) angeordnet sind, die mit der Auswerteschaltung (17) verbunden sind, und dass gegebenenfalls zwischen den Kontaktelektroden (28) und der Auswerteschaltung (17) ein galvanisch getrennter Signalkoppler angeordnet ist.

16. Hautkontakt-Erkennungsvorrichtung nach einem der Ansprüche 9 bis 15, **dadurch gekennzeichnet, dass** der Lichtaustrittsabschnitt (4) und/oder der Lichteintrittsabschnitt (5) mit einer Vorrichtung zur Erzeugung eines Differenzdrucks (33) gegenüber der Umgebung verbunden ist.

17. Hautkontakt-Erkennungsvorrichtung nach einem der Ansprüche 9 bis 16, **dadurch gekennzeichnet, dass** Fotodetektor (9) zumindest für eine dritte Wellenlänge sensitiv ist.

18. Hautkontakt-Erkennungsvorrichtung nach einem der Ansprüche 9 bis 17, **dadurch gekennzeichnet, dass** im Erfassungsbereich (10) des Fotodetektors (9) oder im Fotodetektor (9) ein optisches Filterelement angeordnet ist.

19. Hautkontakt-Erkennungsvorrichtung nach einem der Ansprüche 9 bis 18, **dadurch gekennzeichnet, dass** die Vergleichsschaltung (19) zustandsabhängig ausgebildet ist, insbesondere als state-machine.

20. Hautkontakt-Erkennungsvorrichtung nach einem der Ansprüche 9 bis 19, **dadurch gekennzeichnet, dass** die Referenzdaten der Referenzdatensätze (41, 42) eine Hierarchie aufweisen und/oder dass die Referenzdaten der Referenzdatensätze (41, 42) als Paarungen von Bereichsangaben hinterlegt sind und/oder dass die Referenzdaten der Referenzdatensätze (41, 42) Abweichungsbereiche aufweisen.

21. Hautkontakt-Erkennungsvorrichtung nach einem der Ansprüche 9 bis 20, **dadurch gekennzeichnet, dass** die Sicherungsschaltung (7) einen Umgebungslichtsensor aufweist, welcher mit der Auswerteschaltung (17) verbunden ist, insbesondere mit der Vergleichsschaltung (19).

22. Hautkontakt-Erkennungsvorrichtung nach einem der Ansprüche 9 bis 21, **dadurch gekennzeichnet, dass** die Sicherungsschaltung (7) einen Pulsgenerator aufweist, welcher zur Ansteuerung der Lichtquelle (6) ausgebildet und mit der Vergleichsschaltung verbunden ist.

23. Hautkontakt-Erkennungsvorrichtung nach einem der Ansprüche 9 bis 22, **dadurch gekennzeichnet, dass** die Sicherungsschaltung (7) einen weiteren Lichtsensor aufweist, welcher in Richtung des Lichtaustrittsabschnitts (4) ausgerichtet angeordnet ist und mit der Auswerteschaltung (17) verbunden ist, insbesondere mit der Vergleichsschaltung (19).

24. Lasergerät der Laserklasse 1, umfassend eine Laserquelle mit einer Ansteuerschaltung, und einer Hautkontakt-Erkennungsvorrichtung, wobei die Ansteuerschaltung die Laserquelle nur bei einem Sicherer-Betriebszustand Signal aktiviert, **dadurch gekennzeichnet, dass** die Laserquelle eine Strahlungsleistung größer Laserklasse 1 abgibt, insbesondere dass Strahlungsleistung Klasse 4 abgegeben wird, und dass die Hautkontakt-Erkennungsvorrichtung nach einem der Ansprüche 9 bis 23 ausgebildet ist.

## Claims

1. A method for detecting the approach to and application of a device (24) onto a skin surface (11, 12), having a skin contact detecting device (1), the skin contact detecting device (1) comprising a contact piece (2) having an application surface (3) and a safety circuit (7),
wherein the safety circuit (7) comprises a light source (6), a photodetector (9) and an evaluation circuit (17), said evaluation circuit (17) being connected to the photodetector (9),
wherein
• a first (13) and a second (14) wavelength is emitted from the light source (6) in the direction of the skin surface (11, 12) via a light output section (4) of the application surface (3);
• the first (15) and second (16) wavelength reaching the photodetector (9) via a light input section (5) of the application surface (3) is acquired by the photodetector (9) and is converted into a first (39) and second (40) acquired signal which is proportional to the intensity;
• a first and second time signal profile is constructed by the evaluation circuit (17) for the first (39) and second (40) detected signal;
• wherein a comparator circuit (19) of the evaluation circuit (17) compares the first and second signal profile with the reference data sets (41, 42) stored in a database (43) of the evaluation circuit (17);
• wherein in addition, a safe operating status signal (22) is then emitted by the evaluation circuit (17),
• if the first and second detected signal profile agrees with reference data for first (41) and second (42) acquired signal profiles.

2. The method as claimed in claim 1, **characterized in that** the first and second signal profile is determined by means of a smoothing function.

3. The method as claimed in claim 1 or claim 2, **characterized in that** the comparator circuit (17) carries out a fuzzy comparison and, in addition to agreement, emits at least one second operating status signal, in particular a warning signal.

4. The method as claimed in one of claims 1 to 3, **characterized in that** during the formation of the time signal profiles, a maximum value is found and/or **in that** during the formation of the time signal profiles, a respective trend analysis is carried out.

5. The method as claimed in claim 4, **characterized in that** the trends which are determined are compared with trends stored in the reference data sets (41, 42) or with reference trends determined from the reference data sets.

6. The method as claimed in one of claims 1 to 5, **characterized in that** on applying the device (24) or the contact piece (2) to the skin, almost no first signal (39) is acquired.

7. The method as claimed in one of claims 4 to 6, **characterized in that** the signal profiles which are determined are interpolated linearly from the determined maximum value.

8. The method as claimed in one of claims 1 to 7, **characterized in that** an underpressure or overpressure is applied to the light input section (4) by means of a device for generating a differential pressure (33) and the differential pressure is monitored by means of a pressure measuring device, and when a limiting pressure value for the differential pressure is exceeded, the safe operating status signal (22) is emitted, and upon falling below a limiting pressure value for the differential pressure, the nsafe operating status signal (21) is emitted.

9. A skin contact detecting device (1) for a device (24) to be secured, configured for implementing a method as claimed in one of claims 1 to 8,
comprising
a contact piece (2) with an application surface (3),
a safety circuit (7) with a light source (6), a photodetector (9) and an evaluation circuit (17), said evaluation circuit (17) being connected to the photodetector (9),
wherein the application surface (3) has a light output section (4) and a light input section (5) distinct therefrom,
wherein the light source (6) comprises a main beam direction (8) for the emitted electromagnetic radiation,
said main beam direction (8) being orientated in the direction of the light output section (4),
wherein the photodetector (9) comprises a zone (10) for acquiring electromagnetic radiation,
said acquisition zone (10) being orientated in the direction of the light input section (5), wherein the light source (6) is configured for emitting light of a first (13) and at least a second (14) wavelength
and the photodetector (9) is sensitive to the first (13) and second (14) wavelength and is configured for converting a received first wavelength (15) into a first acquired signal (39) and the received second wavelength (16) into a second acquired signal (40), wherein
the evaluation circuit (17) comprises a database (43), in which reference data sets (41, 42) comprising reference data for acquired first (39) and second (40) signals are stored, wherein the evaluation circuit is configured for forming a first and second time signal profile from the acquired first (39) and second (40) signal,
and wherein further, the evaluation circuit (17) comprises a comparator circuit (19), said comparator circuit (19) being configured for comparing the first and second signal profile with the reference data sets (41, 42) stored in the database (43) and for emitting a safe operating status signal (22).

10. The skin contact detecting device as claimed in claim 9, **characterized in that** the first wavelength (13) is below 470 nm, in particular 450 nm, and/or the second wavelength (14) is in the range 610 nm to 660 nm, in particular 635 nm.

11. The skin contact detecting device as claimed in claim 9 or claim 10, **characterized in that** the contact piece (2) comprises a connecting surface (38) located opposite the application surface (3), wherein the connecting surface (38) is configured for positioning the contact piece (2) on a device (24) to be secured.

12. The skin contact detecting device as claimed in one of claims 9 to 11, **characterized in that** the light output section (4) is connected with the connecting surface (38) via a first aperture (34) in the contact piece (2) and/or **in that** the light input section (5) is connected with the connecting surface (38) via a second aperture (37) in the contact piece (2).

13. The skin contact detecting device as claimed in one of claims 9 to 12, **characterized in that** the light source (6) is formed by at least one light-emitting diode or a laser diode.

14. The skin contact detecting device as claimed in one of claims 9 to 13, **characterized in that** the light source (6) is disposed in the light output section (4).

15. The skin contact detecting device as claimed in one of claims 9 to 14, **characterized in that** at least two contact electrodes (28) are disposed in the application surface (3) which are connected to the evaluation circuit (17) and **in that** if appropriate, a galvanically isolated signal coupler is disposed between the contact electrodes (28) and the evaluation circuit (17).

16. The skin contact detecting device as claimed in one of claims 9 to 15, **characterized in that** the light output section (4) and/or the light input section (5) is/are connected to a device for generating a differential pressure (33) with respect to the environment.

17. The skin contact detecting device as claimed in one of claims 9 to 16, **characterized in that** the photodetector (9) is sensitive to at least a third wavelength.

18. The skin contact detecting device as claimed in one of claims 9 to 17, **characterized in that** an optical filter element is disposed in the acquisition zone (10) of the photodetector (9) or in the photodetector (9).

19. The skin contact detecting device as claimed in one of claims 9 to 18, **characterized in that** the comparator circuit (19) is configured so as to be state-dependent, in particular as a state machine.

20. The skin contact detecting device as claimed in one of claims 9 to 19, **characterized in that** the reference data of the reference data sets (41, 42) comprise a hierarchy and/or **in that** the reference data of the reference data sets (41, 42) are stored as pairs of range data and/or **in that** the reference data of the reference data sets (41, 42) comprise deviation ranges.

21. The skin contact detecting device as claimed in one of claims 9 to 20, **characterized in that** the safety circuit (7) comprises an ambient light sensor which is connected to the evaluation circuit (17), in particular to the comparator circuit (19).

22. The skin contact detecting device as claimed in one of claims 9 to 21, **characterized in that** the safety circuit (7) comprises a pulse generator which is configured so as to control the light source (6) and is connected to the comparator circuit.

23. The skin contact detecting device as claimed in one of claims 9 to 22, **characterized in that** the safety circuit (7) comprises a further light sensor which is aligned in the direction of the light output section (4) and is connected to the evaluation circuit (17), in particular to the comparator circuit (19).

24. A laser device of laser class 1, comprising a laser source with a control circuit, and a skin contact detecting device, wherein the control circuit activates the laser source only upon a Safe operating status signal, **characterized in that** the laser source emits a radiation output greater than laser class 1, and in particular emits class 4 radiation, and **in that** the skin contact detecting device is configured as claimed in one of claims 9 to 23.

## Revendications

1. Procédé de détection d'approche et de l'appui d'un appareil (24) sur une surface de la peau (11, 12), avec un dispositif de détection de contact avec la peau (1), ce dispositif de détection de contact avec la peau (1) comprenant un élément de contact (2) avec une surface d'appui (3) et un circuit de protection (7),
le circuit de protection (7) comprenant une source de lumière (6), un photo-détecteur (9) et un circuit d'analyse (17), ce circuit d'analyse (17) étant relié avec le photo-détecteur (9),
- une première (13) et une deuxième (14) longueur d'onde étant émise en direction de la surface de la peau (11, 12) par la source de lumière (6) par l'intermédiaire d'une portion de sortie de lumière (4) de la surface d'appui (3);
- la première (15) et la deuxième (16) longueur d'onde atteignant le photo-détecteur (9) par l'intermédiaire d'une portion de sortie de lumière (5) de la surface d'appui (3) étant détectée par le photo-détecteur (9) et étant converties en un premier (39) et un deuxième (40) signal détecté dont l'intensité est proportionnelle;
- le circuit d'analyse (17) formant, pour le premier (39) et le deuxième (40) signal détecté, un premier et un deuxième tracé de signal en fonction du temps;
- le circuit de comparaison (19) du circuit d'analyse (17) comparant le premier et le deuxième tracé de signal avec les ensembles de données de référence (41, 42) enregistrés dans une base de données (43) du circuit d'analyse (17);
- le circuit d'analyse (17) émettant en outre un signal d'état de fonctionnement sûr (22),
- lorsque le premier et le deuxième tracé de signal correspond à des ensembles de données pour les premiers (41) et deuxièmes (42) tracés de signaux détecté.

2. Procédé selon la revendication 1, **caractérisé en ce que** le premier et le deuxième tracé de signal est déterminé avec une fonction de lissage.

3. Procédé selon l'une des revendications 1 ou 2, **caractérisé en ce que** le circuit de comparaison (17) effectue une comparaison fine et, en plus de la correspondance, émet au moins un signal d'état de fonctionnement, plus particulièrement un signal d'avertissement.

4. Procédé selon l'une des revendications 1 à 3, **caractérisé en ce que**, lors de la formation des tracés de signaux en fonction du temps, une recherche de valeur maximale est effectuée et/ou **en ce que** lors de la formation des tracés de signaux en fonction du temps, une analyse de tendance est effectuée.

5. Procédé selon la revendication 4, **caractérisé en ce que** les tendances déterminées sont comparées avec les tendances enregistrées dans les ensembles de données de référence (41, 42) ou avec des tendances de référence déterminées à partir des ensembles de données de référence.

6. Procédé selon l'une des revendications 1 à 5, **caractérisé en ce que**, lors de l'appui de l'appareil (24) ou de l'élément de contact (2) sur la peau, presque aucun signal (39) n'est détecté.

7. Procédé selon l'une des revendications 4 à 6, **caractérisé en ce que** les tracés de signaux déterminés sont interpolés linéairement à partir de la valeur maximale déterminée.

8. Procédé selon l'une des revendications 1 à 7, **caractérisé en ce que** la portion d'entrée de lumière (4) est alimentée par un dispositif pour la génération d'une pression différentielle (33) avec une dépression ou une surpression et la pression et la pression différentielle est surveillée par un dispositif de mesure de pression et lors du dépassement d'une valeur limite de pression différentielle, le signal d'état de fonctionnement sûr (22) est émis et lorsqu'une valeur limite de pression différentielle n'est pas atteinte, le signal d'état de fonctionnement non sûr (21) est émis.

9. Dispositif de détection de contact avec la peau (1) pour un appareil (24) à protéger, conçu pour la réalisation d'un procédé selon l'une des revendications 1 à 8, comprenant un élément de contact (2) avec une surface d'appui (3),
un circuit de protection (7) avec une source de lumière (6), un photo-détecteur (9) et un circuit d'analyse (17), ce circuit d'analyse (17) étant relié avec le photo-détecteur (9), la surface d'appui (3) comprenant une portion de sortie de lumière (4) et une portion d'entrée de lumière (5) séparée de celle-ci,
la source de lumière (6) comprenant une direction principale de faisceau (8) du rayonnement électromagnétique émis,
cette direction principale de faisceau (8) étant alignée dans la direction de la portion de sortie de lumière (4),
le photo-détecteur (9) comprenant une zone de détection (10) pour un rayonnement électromagnétique,
cette zone de détection (10) étant alignée en direction de la portion d'entrée de lumière (5),
la source de lumière (6) étant conçue pour l'émission de lumière d'une première (13) et d'au moins une deuxième (14) longueur d'onde
et le photo-détecteur (9) est sensible à la première (13) et à la deuxième (14) longueur d'onde et est conçu pour la conversion d'une première longueur d'onde (15) reçue en un signal détecté (39) et de la deuxième longueur d'onde (16) reçue en un deuxième signal détecté (40),
le circuit d'analyse (17) comprenant une base de données (43) dans laquelle des ensembles de données de référence (41, 42) avec des données de référence pour des premiers (39) et des deuxièmes (40) signaux détectés, le circuit d'analyse étant conçu pour former, à partir du premier (39) et du deuxième (40) signal détecté, un premier et un deuxième tracé de signal en fonction du temps,
et, en outre, le circuit d'analyse (17) comprenant un circuit de comparaison (19),
ce circuit de comparaison (19) étant conçu pour comparer le premier et le deuxième tracé de signal en fonction du temps avec les ensembles de données de référence (41, 42) enregistrés dans la base de données (43) et pour émettre un signal d'état de fonctionnement sûr (22).

10. Dispositif de détection de contact avec la peau selon la revendication 9, **caractérisé en ce que** la première longueur d'onde (13) est inférieure à 470 nm, plus particulièrement 450 nm et/ou **en ce que** la deuxième longueur d'onde (14) est de l'ordre de 610 nm à 660 nm, plus particulièrement 635 nm.

11. Dispositif de détection de contact avec la peau selon l'une des revendications 9 ou 10, **caractérisé en ce que** l'élément de contact (2) comprend une surface de raccordement (38) opposée à la surface de contact (3), la surface de raccordement (38) étant conçue pour la disposition de l'élément de contact (2) sur un appareil à protéger (24).

12. Dispositif de détection de contact avec la peau selon l'une des revendications 9 à 11, **caractérisé en ce que** la portion de sortie de lumière (4) est reliée, par l'intermédiaire d'un premier passage (34) dans l'élément de contact (2) et/ou **en ce que** la portion d'entrée de lumière (5) est reliée, par l'intermédiaire d'un deuxième passage (37), conçu comme un guide d'onde, dans l'élément de contact (2), avec la surface de raccordement (38).

13. Dispositif de détection de contact avec la peau selon l'une des revendications 9 à 12, **caractérisé en ce que** la source de lumière (6) est constituée d'au moins une diode luminescente ou une diode laser.

14. Dispositif de détection de contact avec la peau selon l'une des revendications 9 à 13, **caractérisé en ce que** la source de lumière (6) est disposée dans la portion de sortie de lumière (4).

15. Dispositif de détection de contact avec la peau selon l'une des revendications 9 à 14, **caractérisé en ce que**, dans la surface d'appui (3) se trouvent au moins deux électrodes de contact (28) qui sont reliées avec le circuit d'analyse (17) et **en ce que**, le cas échéant, entre les électrodes de contact (28) et le circuit d'analyse (17), se trouve un coupleur de signaux séparé galvaniquement.

16. Dispositif de détection de contact avec la peau selon l'une des revendications 9 à 15, **caractérisé en ce que** la portion de sortie de lumière (4) et/ou la portion d'entrée de lumière (5) est reliée avec un dispositif de génération d'une pression différentielle (33) par rapport à l'environnement.

17. Dispositif de détection de contact avec la peau selon l'une des revendications 9 à 16, **caractérisé en ce que** le photo-détecteur (9) est sensible à au moins une troisième longueur d'onde.

18. Dispositif de détection de contact avec la peau selon l'une des revendications 9 à 17, **caractérisé en ce que**, dans la zone de détection (10) du photo-détecteur (9) ou dans le photo-détecteur (9), se trouve un élément de filtre optique.

19. Dispositif de détection de contact avec la peau selon l'une des revendications 9 à 18, **caractérisé en ce que** le circuit de comparaison (19) est conçu en fonction de l'état, plus particulièrement sous la forme d'un automate fini.

20. Dispositif de détection de contact avec la peau selon l'une des revendications 9 à 19, **caractérisé en ce que** les données de référence des ensembles de données de référence (41, 42) présentent une hiérarchie et/ou **en ce que** les données de référence des ensembles de données de référence (41, 42) sont enregistrées sous la forme d'accouplements d'informations de zones et/ou **en ce que** les données de référence des ensembles de données de référence (41, 42) présentent des plages de variation.

21. Dispositif de détection de contact avec la peau selon l'une des revendications 9 à 20, **caractérisé en ce que** le circuit de protection (7) comprend un capteur de lumière ambiante qui est relié avec le circuit d'analyse (17), plus particulièrement avec le circuit de comparaison (19).

22. Dispositif de détection de contact avec la peau selon l'une des revendications 9 à 21, **caractérisé en ce que** le circuit de protection (7) comprend un générateur d'impulsions qui est conçu pour contrôler la source de lumière (6) et qui est relié au circuit de comparaison.

23. Dispositif de détection de contact avec la peau selon l'une des revendications 9 à 22, **caractérisé en ce que** le circuit de protection (7) comprend un capteur de lumière supplémentaire qui est aligné en direction de la portion de sortie de lumière (4) et relié au circuit d'analyse (17), plus particulièrement au circuit de comparaison (19).

24. Appareil laser de classe laser 1, comprend une source laser avec un circuit de commande et un dispositif de détection de contact avec la peau, le circuit de commande activant la source laser uniquement en présence d'un signal d'état de fonctionnement sûr, **caractérisé en ce que** la source laser génère une puissance de rayonnement supérieure à la classe de laser 1, plus particulièrement **en ce qu'**une puissance de classe 4 est générée et **en ce que** le dispositif de détection de contact avec la peau est conçu selon l'une des revendications 9 à 23.
